# EUROPEAN PATENT SPECIFICATION

(11) **EP 1 709 900 B1**
(45) Date of publication and mention of the grant of the patent: **23.03.2011**
(21) Application number: 05709309.8
(22) Date of filing: 25.01.2005
(51) Int. Cl.: A61B 1/00, A61B 17/00

(54) **ENDOSCOPE TREATMENT SYSTEM**
ENDOSKOPBEHANDLUNGSSYSTEM
SYSTEME DE TRAITEMENT ENDOSCOPIQUE

(30) Priority: 27.01.2004 JP 2004018315; 28.01.2004 JP 2004019823
(43) Date of publication of application: 11.10.2006
(73) Proprietor: Olympus Corporation, Tokyo 151-0072 (JP)
(72) Inventor: KAJI, Kunihide, 1920023 (JP); SUZUKI, Keita, 1900013 (JP); OKADA, Tsutomu, 1900012 (JP); NAKANO, Tadahiro, 1920001 (JP)
(74) Representative: von Hellfeld, Axel
(86) International application number: PCT/JP2005/000908
(87) International publication number: WO 2005/070282

(56) References cited:
- JP-A- 6 054 801
- JP-A- 56 008 028
- JP-A- 2000 014 631
- JP-U- 60 184 513
- US-A- 6 059 719

## Description

### TECHNICAL FIELD

The present invention relates to an endoscope treatment system.

### BACKGROUND ART

When carrying out a procedure at a specific site or examining a body tissue within the body using an endoscope that is manipulated from outside the body, it is typically the case that a variety of procedure instruments such as a needle scalpel or gripping forceps are used sequentially. In this case, it has been the conventional practice to insert the selected instrument into the body by passing it through a channel that is provided within the inserted portion of the endoscope. Once the specific procedure has been performed, the instrument is then withdrawn from the body, and another instrument is inserted into the channel, with this process repeated accordingly.

When inserting this type of instrument into the channel, considerable care must be used when inserting a long instrument via the forceps port into the narrow channel so that it takes time and requires close attention. For this reason, an endoscope provided with an inserting and withdrawing device for automatically carrying out the operation of inserting instruments into and withdrawing instruments from the channel was proposed (see, for example, Patent Document 1, below).

However, in the endoscope treatment system disclosed in Patent Document 1, conventional instruments can be used without modification. As a result, it is necessary for the assistant to assist in the procedure by providing support with his hand when inserting an instrument into or withdrawing an instrument from the endoscope. Moreover, because manipulation of the instruments is not carried out by a technician, but rather by an assistant, it still takes time and it is difficult to manipulate.

On the other hand, a rigid forceps to which the inserted part of an instrument and a treatment unit are detachably connected (see Patent Document 2) and an instrument in which a connector is provided that enables attachment or detachment of the inserted parts of respective instruments (see Patent Document 3) have been proposed.

In particular, the instrument disclosed in Patent Document 3 enables a treatment unit to be inserted into the channel quickly by attaching a detachable treatment unit to the inserted part of the instrument.

However, when attaching a treatment unit having a treatment part to the inserted part of an instrument, in the technique disclosed in Patent Documents 2 and 3, it is necessary to carry out the action of connecting the treatment unit and the inserted part of the instrument, and the action of connecting the treatment part and the inserted part of the instrument as separate operations and from different directions and, therefore, it is not sufficient to quickly attach the treatment unit.
[Patent Document 1] Japanese Unexamined Patent Application, First Publication No. 57-117823 (FIG. 1)
[Patent Document 2] U.S. Patent No. 5,368,606 (FIG. 3)
[Patent Document 3] U.S. Patent No. 5,782,748 (FIGS. 7, 8 and 9)

JP 06/054801 discloses an endoscope provided with a multifunction treating implement having a small size. A treatment unit consists of a curvable arm part and effectors attached to the arm part so as to be freely attachable, detachable, and changeable. Further, driving means for operating the treatment unit and a mechanism for controlling the driving means and for connecting and separating the arm part and the effectors is provided. The arm part is connected to the effector via a magnetic connection, and, when the art part is bent so as to allow a distal end of the arm part to enter a changing port, the magnetic connection may be released and the effector may be dissociated from the arm part and suction draws the effector proximally through the treatment unit. Thereby, the arm part may be inserted into a different channel of the endoscope, and a different effector may be selected and coupled to the arm part.

### DISCLOSURE OF THE INVENTION

The present invention takes such circumstances into consideration, with an object of providing an endoscope treatment system capable of facilitating insertion and extraction operation of a treatment part and carrying out manipulation in shorter period of time and more easily.

Further, an object of the present invention is to provide an endoscope treatment system enabling the treatment unit to be connected by means of unidirectional continuous operation, and capable of achieving manipulation in shorter period of time and more easily.

To solve the above problems, an endoscope according to claim 1 is provided.

The endoscope treatment system according to the present invention is characterized by including an endoscope having an inserted part; a channel provided along the inserted part; a treatment unit which can be inserted into the channel and has a treatment part at its front end; a treatment unit driving mechanism for moving the treatment unit inside the channel; and a locking member provided near the front end of the channel in the inserted part, for locking the treatment unit.

As a result, the treatment unit can be held at a specific position near the front end of the channel of the inserted part, and it is possible to prevent the treatment unit from coming free from the front end of the channel.

In addition, the endoscope treatment system according to the present invention is characterized in that the treatment unit driving mechanism is an advance driving mechanism for advancing the treatment unit within the channel toward the front end of the channel.

By using the advance driving mechanism to advance a treatment unit which has been inserted into the channel, it is possible to pass the treatment unit through the channel and move it toward the front end side thereof. In addition, by locking the treatment unit with the advance blocking part, it is possible to prevent the treatment unit from coming free from the front end of the channel.

Further, in the endoscope treatment system, the endoscope treatment system according to the present invention is characterized in that the treatment unit is provided with a treatment part driving mechanism for receiving the advance/retract driving force and driving the treatment part, and a connecting mechanism for connecting the front end of the advance driving mechanism and the base end side of the treatment part driving mechanism, by advancing the advance driving mechanism with the treatment unit locked by the advance blocking part.

In this endoscope treatment system, when the advance driving mechanism is advanced with the treatment unit locked by the advance blocking part, the front end of the advance driving mechanism and the base end side of the treatment part driving mechanism can be connected by the connecting mechanism.

Further, in the endoscope treatment system, the endoscope treatment system according to the present invention is characterized in that the advance driving mechanism is provided with a tubular operation tube, and an operation line that is disposed to enable advance and retraction along the axial direction of the operation tube and is for connecting to the treatment part driving mechanism by a connecting mechanism; and that the treatment unit be provided with an operation tube locking part for locking the operation tube.

In this endoscope treatment system, by moving the operation tube and the operation line, together with the treatment unit, toward the front end side of the channel until the operation tube locking part is locked by the advance blocking part, it is possible to place the operation tube in a locked state with respect to the channel using the operation tube locking part. Accordingly, by moving the operation line relative to the operation tube, the treatment part driving mechanism can be connected to the operation line by the connecting mechanism.

In the endoscope treatment system, the endoscope treatment system according to the present invention is characterized in that the connecting mechanism is provided with a coupler main body formed in the shape of a rod projecting out from the base end side of the treatment unit; a round member disposed to the side edge of the operation tube of the coupler main body and having a diameter that is larger than the outer diameter of the coupler main body; a concave part that is disposed to the front end of the operation line and is for engaging with the round member; and a slit for guiding the round member into the concave part, in which the width of the opening is smaller than the diameter of the round member and which can be elastically deformed to enable insertion of the round member.

In this endoscope treatment system, when the concave part is pressed into the round member, the round member enters into the concave part as the slit is widened. The round part engages in the concave part, and the width of the slit opening returns to the opening width that is smaller than the round member. As a result, the concave part is prevented from falling out from the slit, thereby enabling coupling of the treatment unit and the operation wire part.

In the endoscope treatment system, the present invention's endoscope treatment system is characterized in that the advance blocking part is detachable with respect the front end of the channel.

With this endoscope treatment system, it is possible to attach an advance blocking part to the channel of a conventional endoscope as well, so that the treatment unit and the advance driving mechanism can be easily connected inside the channel.

In the endoscope treatment system, the present invention's endoscope treatment system is characterized by including an elastic member that can freely expand and contract in the axial direction, between the operation tube locking part and the advance blocking part.

In this endoscope treatment system, the operation tube locking part is locked via the elastic member by the advance blocking part, and the elastic member can be compressed in the axial direction by moving the operation tube and the operation line further toward the front end of the channel. Accordingly, by setting the treatment unit to a suitable length in advance, the treatment unit can be projected out from the front end of the channel to an extent permitted by the elastic deformation limits of the elastic member, and a procedure can be carried out at a site separated from the front end of the inserted part of the endoscope in the same manner as when employing conventional procedure instruments.

In the endoscope treatment system, the present invention's endoscope treatment system is characterized in that at least one of either a tube-side concave part or a tube-side convex part is disposed to the front end of the operation tube, and at least one of either a locking-side convex part or a locking-side concave part that can engage with at least one of either the tube-side concave part or the tube-side convex part is disposed to the operation tube locking part.

By engaging at least one of either a tube-side concave part and a locking-side convex part, or a tube-side convex part and a locking-side concave part, not only can the operation tube and the operation tube locking part be engaged along the axial direction, but it is also possible to stop axial rotation. As a result, both members can be rotated together, so that the operation of rotating the treatment unit can be carried out easily.

Further, the present invention's endoscope treatment system is characterized in that the treatment unit has a treatment part main body for supporting the treatment part; the locking member is a restricting member that can restrict advancing or retracting movement of the treatment unit inside the channel; and the treatment unit driving mechanism is an advance/retract driving mechanism for advancing or retracting the treatment unit between the base end side and the front end side of the channel, and for driving the treatment part by advancing or retracting the treatment part relative to the channel and the treatment part main body in a state where the advance or retraction of the treatment part main body is restricted by the restricting member.

In this endoscope treatment system, the treatment unit is inserted into the channel and can be advanced and retracted within the channel by the advance/retract driving mechanism. In addition, the treatment part can be operated by manipulating the advance/retract driving mechanism and the restricting means together.

In addition, in the endoscope treatment system, the present invention's endoscope treatment system is characterized in that the restricting member is provided with a convex part that can recess inward in the radial direction of the channel, and a recessing mechanism for recessing this convex part.

In the endoscope treatment system, the recessing mechanism is employed to project the convex part out from within the channel, so that the advance and retract movement of the treatment unit can be stopped and the treatment part can be manipulated.

In addition, in the endoscope treatment system, the present invention's endoscope treatment system is characterized by including a blocking mechanism for blocking the action of the treatment part during advancing and retracting movement of the treatment unit.

In this endoscope treatment system, it is possible to prevent the treatment unit from accidentally operating during advancing and retracting of the treatment unit inside the channel.

In addition, in the endoscope treatment system, the present invention's endoscope treatment system is characterized in that the treatment unit is formed so that it is detachable with the advance/retract driving mechanism.

When carrying out a plurality of different procedures, this endoscope treatment system does not require that the entire treatment part be exchanged as in the case of conventional endoscope treatment system. Rather, only the treatment unit need be exchanged in the case where carrying out a plurality of different procedures.

In addition, in the endoscope treatment system, the present invention's endoscope treatment system is characterized in that the advance/retract driving mechanism is provided with an operation line that can advance and retract within the channel and is connected to the treatment unit, and an advance/retract mechanism for advancing and retracting the operation line; the treatment unit is provided with a treatment part driving mechanism that is supported by the treatment part main body and connected to the procedure line part, and is for communicating the advancing/retracting action of the operation line with respect to the treatment part main body, to the treatment part; and there is provided a locking mechanism that can rotate around a center axis in a state wherein the treatment part driving mechanism and the operation line are mutually locked.

In this endoscopic treatment unit, the operation line is advanced or retracted using the advance/retract mechanism to advance or retract the treatment unit inside the channel. In addition, by means of the treatment unit main body coming into contract with the restricting member, the operation line can be advanced or retracted relative to the treatment unit main body, and the treatment part can be manipulated via the treatment part driving mechanism.

In addition, even if the treatment unit must be rotated around its axis during a procedure, it is possible to transmit this rotation to the treatment unit by rotating the operation line around the axis, and thereby carry out the procedure more easily and certainly.

In the endoscope treatment system, the endoscope treatment system according to the present invention is characterized in that the outer peripheral surface of the operation line is covered with a helical member.

In this endoscope treatment system the likelihood that the operation line will buckle is decreased, even if a load is placed in the compressing direction on the operation line.

Further, in the endoscope treatment system, the endoscope treatment system according to the present invention is characterized by including an extension part that comes into contact with the restricting member, is connected to the treatment unit and the advance/retract driving mechanism, and is formed to have a length capable of projecting the treatment unit out from the front end of the channel.

By disposing an extension part of the desired length in this endoscope treatment system, it is possible to project the treatment unit out from the front end of the channel by the desired amount, and to carry out a procedure to a lesion site located away from the front end of the channel.

In addition, in the endoscope treatment system, the endoscope treatment system according to the present invention is characterized by including a projecting mechanism capable of projecting the convex part along with a portion of the channel outward from the front end of the channel.

By actuating the projecting mechanism in this endoscope treatment system, it is possible to project the treatment unit out from the front end of the channel by the desired amount, and to carry out a procedure to a lesion site located away from the front end of the channel.

The present invention enables the treatment unit, and the operation tube and operation line, to be connected by means of a unidirectional continuous operation, and makes it possible to manipulate the treatment unit, to carry out a procedure in shorter period of time, more easily.

Moreover, with the present invention, the treatment part can be manipulated even without providing to the end of a procedure instrument an operator for manipulating the treatment part, as has been required conventionally. Accordingly, the technician can carry out the procedure using the procedure instrument while manipulating the endoscope, and the time required for the procedure can be reduced. In addition, since a coil sheath such as employed in the conventional art is not required for the procedure instrument, a more compact housing can be accomplished.

### BRIEF DESCRIPTION OF THE DRAWINGS

FIG. 1 is a side view including a partial cross-section showing the endoscope treatment system according to a first embodiment of the present invention.
FIG. 2 is a side view including a partial cross-section showing essential parts of the endoscope treatment system according to a first embodiment of the present invention.
FIG. 3 is a cross-sectional view showing the front end of the operation wire and the operation tube in the endoscope treatment system according to a first embodiment of the present invention.
FIG. 4 is a side view including a partial cross-section showing essential parts of the endoscope treatment system according to a first embodiment of the present invention.
FIG. 5 is a side view including a partial cross-section showing the endoscope treatment system according to a first embodiment of the present invention.
FIG. 6 is a side view including a partial cross-section showing the endoscope treatment system according to a second embodiment of the present invention.
FIG. 7 is a cross-sectional view showing the treatment unit of the endoscope treatment system according to a second embodiment of the present invention.
FIG. 8 is a side view including a partial cross-section showing essential parts of the endoscope treatment system according to a second embodiment of the present invention.
FIG. 9 is a side view of an essential part including a partial cross-section showing the endoscope treatment system according to a third embodiment of the present invention.
FIG. 10 is a view along the direction of arrow A in FIG. 9.
FIG. 11 is a side view including a partial cross-section showing the endoscope treatment system according to a fourth embodiment of the present invention.
FIG. 12 is a side view including a partial cross-section showing the endoscope treatment system according to a fifth embodiment of the present invention.
FIG. 13 is a side view including a partial cross-section showing the endoscope treatment system according to a fifth embodiment of the present invention.
FIG. 14 is a side view including a partial cross-section showing essential parts of the endoscope treatment system according to a fifth embodiment of the present invention.
FIG. 15 is a side view including a partial cross-section showing essential parts of the endoscope treatment system according to a sixth embodiment of the present invention.
FIG. 16A is a side view showing essential parts of the endoscope treatment system according to a seventh embodiment of the present invention.
FIG. 16B is a side view including a partial cross-section showing essential parts of the endoscope treatment system according to a seventh embodiment of the present invention.
FIG. 17A is an explanatory view showing the operating method of the endoscope treatment system according to a seventh embodiment of the present invention, showing the state prior to engagement of the treatment unit and the operation wire.
FIG 17B is an explanatory view showing the operating method of the endoscope treatment system according to a seventh embodiment of the present invention, showing the state when engaging the treatment unit and the operation wire.
FIG. 17C is an explanatory view showing the operating method of the endoscope treatment system according to a seventh embodiment of the present invention, showing the state prior to engagement of the treatment unit and the operation wire.
FIG. 18 is a side view showing the endoscope treatment system according to an eighth embodiment of the present invention
FIG. 19 is a cross-sectional view in which part A in FIG. 18 is enlarged.
FIG. 20 is a cross-sectional view in which part B in FIG. 18 is enlarged.
FIG. 21 is an explanatory view showing the operating method for the endoscope treatment system according to an eighth embodiment of the present invention.
FIG. 22 is a side view including a partial cross-section showing essential components of the endoscope treatment system according to a ninth embodiment of the present invention.
FIG. 23 is a side view including a partial cross-section showing essential components of the endoscope treatment system according to a tenth embodiment of the present invention.
FIG. 24 is a cross-sectional view showing the rotation mechanism of the endoscope treatment system according to a tenth embodiment of the present invention.
FIG. 25 is a side view showing essential components of the endoscope treatment system according to an eleventh embodiment of the present invention.
FIG. 26 is a side view including a partial cross-section showing essential components of the endoscope treatment system according to an eleventh embodiment of the present invention.
FIG. 27 is a side view showing essential components of the endoscope treatment system according to a modification of an eleventh embodiment of the present invention.
FIG. 28A is a cross-sectional view showing essential parts of the endoscope treatment system according a twelfth embodiment of the present invention, showing the state prior to locking the treatment unit.
FIG. 28B is a cross-sectional view showing essential parts of the endoscope treatment system according a twelfth embodiment of the present invention, showing the state after the treatment unit has been locked.
FIG. 29 is a side view showing essential parts of the endoscope treatment system according to a modification of the embodiments of the present invention.
FIG. 30 is a side view showing essential parts of the endoscope treatment system according to another modification of the embodiments of the present invention.

### BEST MODE FOR CARRYING OUT THE INVENTION

Next, Embodiments 1 through 12 of the present invention will be explained with reference to the figures.

### [Embodiment 1]

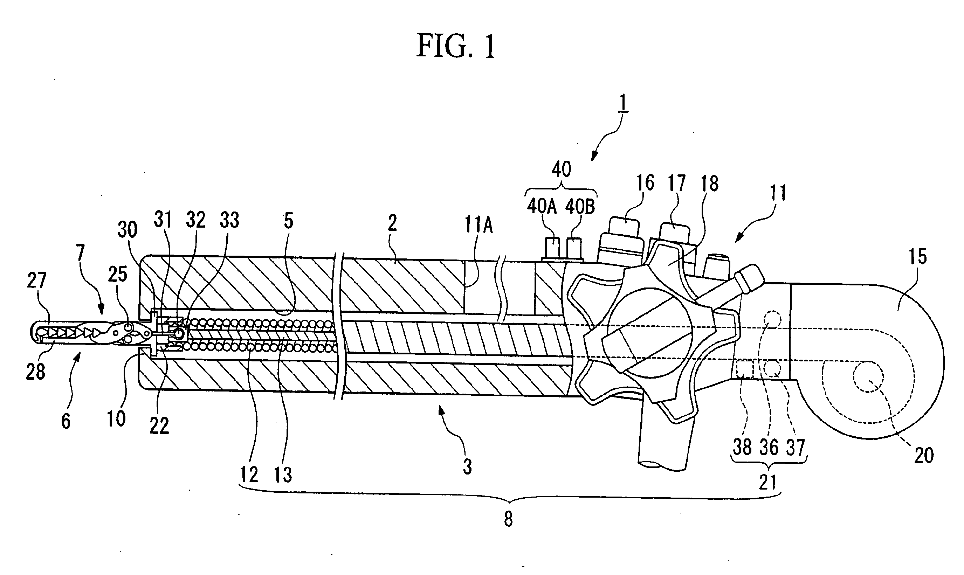

A first embodiment of the present invention will now be explained with reference to FIGS. 1 through 5.

Referring to FIG 1, the endoscope treatment system 1 according to this embodiment is provided with an endoscope 3, having an inserted part 2; a channel 5 provided along the inserted part 2; a treatment unit 7 that has a treatment part 6 at its front end and can be inserted into the channel 5; and an advance driving means 8 that can freely advance and retract in a direction parallel to channel 5, for advancing the treatment unit 7 inside the channel 5. An advance blocking part 10 is disposed to the front end of the channel 5 for preventing the forward movement of the treatment unit 7 by locking the treatment unit 7 when it is being advanced.

An operator 11 is connected to the base end of the inserted part 2. A housing 15 that can house the base end side of an operation tube 12 and an operation wire (operation line) 13, which are explained below, is connected to the base end of the operator 11. The channel 5 is disposed so as to connect the inside of the inserted part 2 and the operator 11, and is connected to the open end of the housing 15.

A gas/water relay button 16, suction button 17, and operating knob 18 are disposed to the operator 11. In addition, a port 11A is formed in the operator 11 connected along the channel 5, this port 11A enabling insertion of the treatment unit 7 into the channel 5.

The housing 15 is cylindrical in shape and houses the operation tube 12 and the operation wire 13 by rolling them around a center part, and is provided with a core member 20 that can rotate around its center axis.

The advance blocking part 10 is formed projecting inward in the radial direction from the inner peripheral surface of the channel 5 at the front end of the inserted part 2.

The advance driving means 8 is provided with the tubular operation tube 12 that is wrapped in a coil; the operation wire 13 that is disposed to enable advancing and retracting in the axial direction inside the operation tube 12; and a driving mechanism 21 for forward driving of the operation tube 12 and the operation wire 13.

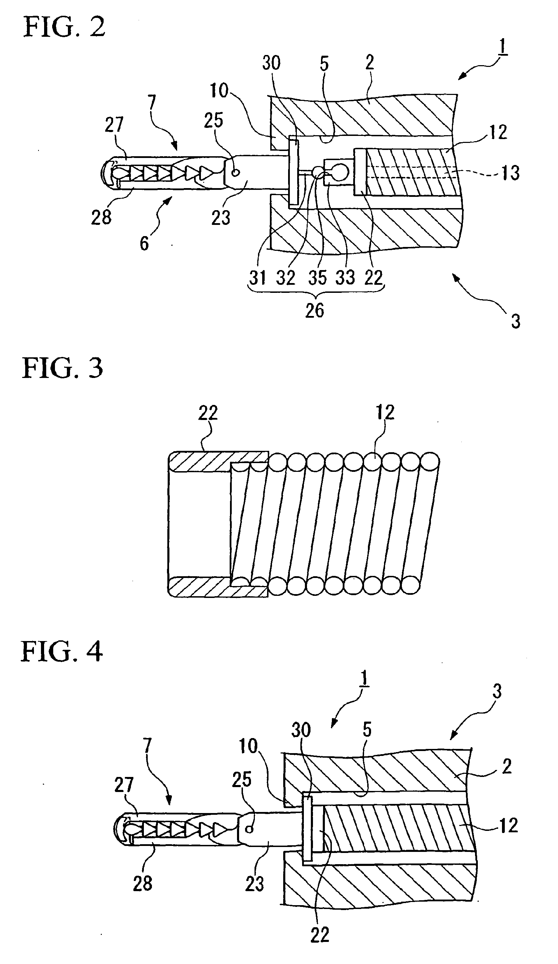

As shown in FIG. 3, a base 22 having the same diameter as the operation tube 12 is disposed to the front end of the operation tube 12.

As shown best in FIG. 2, the treatment unit 7 is provided with a cover member 23 which is the main body and is cylindrical shape; a treatment part driving mechanism 25 for receiving the advance/retract driving force from the operation wire 13 to activate the treatment part 6; and a connecting means 26 for connecting the front end of the operation wire 13 and the base end side of the treatment part driving mechanism 25 by advancing the operation wire 13 under the condition where the treatment unit 7 has been locked by the advance blocking part 10.

The treatment part 6 is provided with paired forceps pieces 27 and 28 for carrying out a procedure that are disposed to the front end side of the cover member 23.

The paired forceps pieces 27 and 28 can be opened and closed by advancing or retracting the operation wire 13 relative to the operation tube 12 using a procedure operator part not shown.

A flange-shaped operation tube locking part 30 is provided to the base end of the cover member 23 for locking the operation tube 12, operation tube locking part 30 having a diameter such that it can come into contact with the base 22.

The connecting means 26 is provided with a coupler main body 31 that is formed projecting out in the form of a rod from the base end of the treatment part driving mechanism 25 of the treatment unit 7; a round member 32 that is disposed to the end of the coupler main body 31 on the operation tube 12 side and has a diameter that is larger than the outer diameter of the coupler main body 31; a concave part 33 that is disposed to the front end of the operation wire 13 and can engage with the round member 32; and a slit 35 that guides the round member 32 into the concave part 33 and has an opening width that is smaller then the diameter of the round member 32 and is formed to elastically deform so that round member 32 can be inserted.

A driving mechanism 21 is detachably connected to the endoscope 3 on the operator 11 base end side, and is provided with paired rollers 36 and 37 that are attached facing one another to enable free rotation and which apply pressure along the radial direction of the operation tube 12; a driver 38 consisting of a motor or the like that rotates these paired rollers 36 and 37 simultaneously at the same speed; and a controller, not shown in the figures, for controlling activation of the driver 38.

The controller is linked with a switch 40 which is provided to the operator 11, with drive control carried out by manipulation of this switch 40.

The switch 40 is disposed to the operator 11, and is provided with an advance switch 40A which, when depressed, causes both the operation tube 12 and the operation wire 13 to move toward the front end side of the channel 5; and a retract switch 40B which, when depressed, causes both the operation tube 12 and the operation wire 13 to move toward the base end side of the channel 5. When both of the switches 40A,40B are released, the driver 38 is stopped.

Next, the operating method using the endoscope treatment system 1 of this embodiment consisting of the above design will be explained below.

First, the treatment unit 7 is inserted into the port 11A, and the inserted part 2 of the endoscope 3 assembled into the state as shown in FIG. 1 is inserted into the body cavity. The advance switch 40A is depressed and the driver 38 is actuated, so that the paired rollers 36 and 37 are rotated in the direction that sends the operation tube 12 and the operation wire 13 into the inserted part 2. As a result, the operation tube 12 and the operation wire 13, which are held under pressure between the paired rollers 36 and 37, are sent out from the housing 15 into the channel 5.

Once the ends of the operation tube 12 and the operation wire 13 are moved to the position of the treatment unit 7, the base 22 at the front end of the operation tube 12 comes into contact with the operation tube locking part 30 of the treatment unit 7.

The treatment unit 7 is moved along with the operation tube 12 and the operation wire 13 toward the front end side of the channel 5, and, as shown in FIG. 4, the operation tube locking part 30 is locked by the advance blocking part 10 at the front end of the channel 5, thereby halting the treatment unit 7.

The advance switch 40A is released, stopping movement of the operation tube 12 and the operation wire 13, after which the operation wire 13 is advanced toward the front end side of the channel 5 as shown in FIG. 2 using a procedure operator not shown in the figures. Since the operation wire 13 advances relative to the cover member 23 at this time, the round member 32 at the base end of the coupler main body 31 of the connecting means 26 elastically deforms the slit 35 in the concave part 33 at the front end of the operation wire 13, and is pushed inside the slit 35 as the opening widens. Once the round member 32 is pushed through the slit 35, the width of the opening of the slit 35 contracts back to a width which is smaller than the diameter of the round member 32, thereby engaging the coupler main body 31.

The round member 32 is prevented from separating from the concave part 33, thus coupling the treatment part driving mechanism 25 and the operation wire 13.

Note that, prior to inserting the treatment unit 7 into the port 11A, it is also acceptable for the operator to manually directly connect the treatment part driving mechanism 25 and the operation wire 13 and then insert the treatment unit 7 into the channel 5 and move it toward the front end thereof.

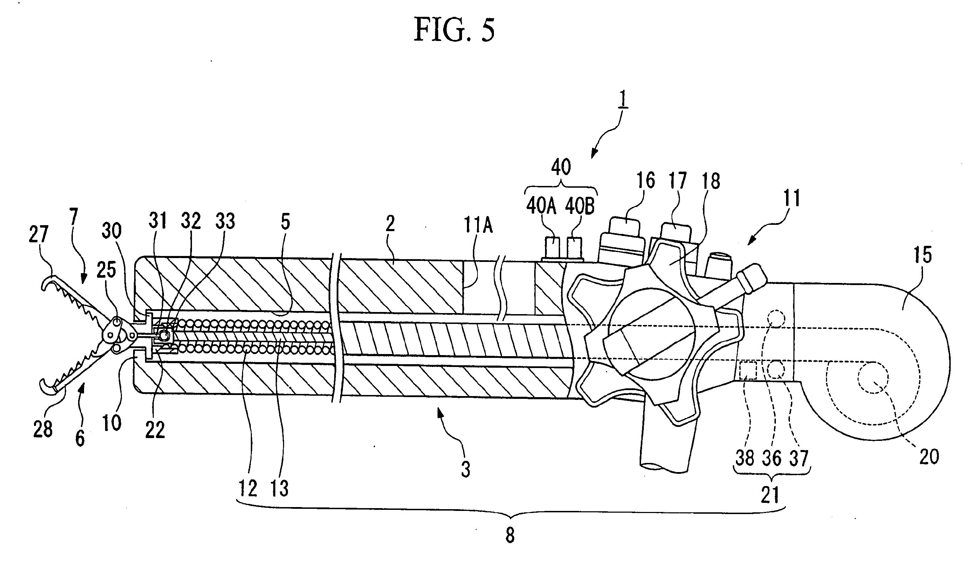

When carrying out a procedure using the treatment part 6, the operation wire 13 is advanced and retracted along the channel 5 by operating a treatment part operator not shown in the figures. This movement is transmitted to the treatment part driving mechanism 25 via the connecting means 26 and is converted into the opening/closing operation of the paired forceps pieces 27 and 28, opening the paired forceps pieces 27 and 28 as shown in FIG. 5. In the case where gripping a lesion site, the lesion site can be gripped by retracting the operation wire 13 and causing the paired forceps pieces 27 and 28 to close.

After completing the procedure using the treatment part 6, the retract switch 40B is depressed and the driver 38 is actuated, causing the paired rollers 36 and 37 to rotate in the direction opposite the direction of insertion. At this time, the treatment unit 7 moves together with the operation tube 12 and the operation wire 13 within the channel 5 toward the base end side of the inserted part 2. At this time, the core member 20 of housing 15 is pushed by the operation tube 12 and the operation wire 13, causing it to rotate, so that the operation tube 12 and the operation wire 13 are wound around the core member 20.

Once the treatment unit 7 arrives at the position of the port 11 A, the retract switch 40B is released, halting the driver 38. The concave part 33 and the round member 32 are separated, and the treatment unit 7 is released from the operation wire 13 and recovered.

In the case where a procedure is to be carried out here using another procedure instrument, a separate procedure instrument is inserted into the channel 5 in the same manner as for the treatment unit 7. The driver 38 is then actuated in the same manner as described above, to move the operation tube 12 and the operation wire 13 toward the front end of the channel 5. After connection of the operation tube 12 and the operation wire 13, the new procedure is then carried out.

To complete the procedure, the driver 38 is actuated and the operation tube 12 and the operation wire 13 are housed inside the housing 15.

In this endoscope treatment system 1, the operation tube 12 and the operation wire 13 are moved together with the treatment unit 7 toward the front end side of the channel 5 until the operation tube locking part 30 is locked by the advance blocking part 10. As a result, the operation tube 12 enters a locked state with respect to the channel 5 due to the operation tube locking part 30. By moving the operation wire 13 relative to the operation tube 12 at this time, the round member 32 is pushed against the concave part 33 of the operation wire 13, and is introduced into the concave part 33 as the slit 35 widens. As a result, it is possible to achieve optimal coupling of the treatment unit 7 and the operation wire 13.

In addition, by locking the operation tube 12 with the advance blocking part 10, it is possible to prevent the treatment unit 7 from pulling free from the front end of the channel 5.

### [Embodiment 2]

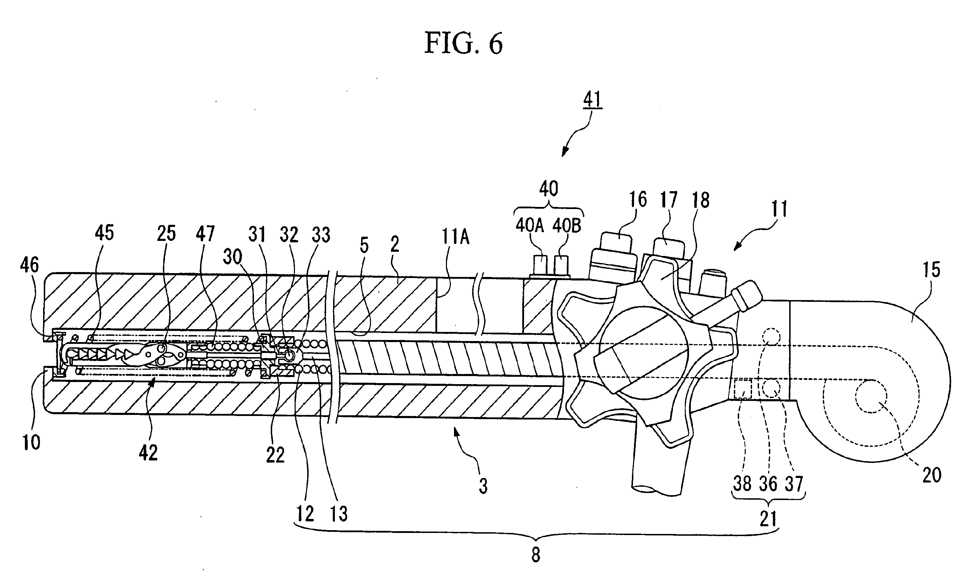

A second embodiment of the present invention will now be explained with reference to FIGS. 6 through 8.

Note that structural elements that are equivalent to those of the above-described first embodiment will be assigned the same numeric symbol and an explanation thereof will be omitted here.

The difference between the first and second embodiments is that in the endoscope treatment system 41 according to the second embodiment, a spring (elastic element) 45 capable of freely extending and compressing along the axial direction is disposed to a position that is between the operation tube locking part 30 and the advance blocking part 10 of the treatment unit 42.

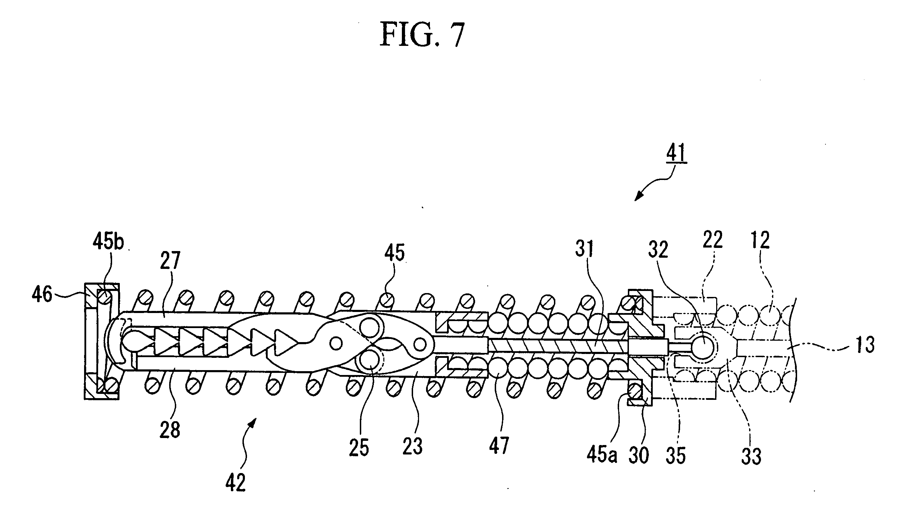

As shown in FIGS. 6 and 7, the base end 45a of the spring 45 is connected to the lateral surface of the front end side of the operation tube locking part 30. A cylindrically-shaped spring short tube 46 is disposed to the front end 45b of the spring 45.

The length in the axial direction of spring 45 when in its natural state is set based on the distance from the front end of the inserted part 2 of the endoscope 3 to the lesion site that is to be treated.

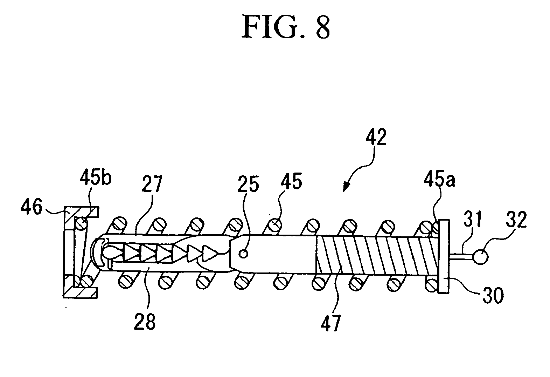

As shown in FIG. 8, a bendable coil-shaped sheath 47 is connected to the base end of the cover member 23 of the treatment unit 42, and the operation tube locking part 30 and the base end of the sheath 47 are connected. The length of the sheath 47 is formed based on the length of the spring 45 when in its natural state.

The coupler main body 31 of the connecting means 26 is formed to have a length that extends based on the length of the sheath 47.

Next, the operating method using an endoscope treatment system 41 according to this embodiment consisting of the above-described structure will be explained below.

First, the inserted part 2 of the endoscope 3 assembled into the state as shown in FIG. 6 is inserted into the body cavity. Next, the treatment unit 42 is inserted into the port 11A, and the operation tube 12 and the operation wire 13 are relayed from the housing 15 into the channel 5 in the same manner as in the first embodiment.

When the treatment unit 42 is moved together with the operation tube 12 and the operation wire 13 toward the front end side of the channel 5, the spring short tube 46 is locked by the advance blocking part 10 at the front end of the channel 5, and this deterrent force is communicated to the operation tube locking part 30 via the spring 45, halting the treatment unit 42.

The advance switch 40A is released, halting the movement of the operation tube 12 and the operation wire 13, after which the operation wire 13 is advanced toward the front end side of the channel 5 using an operation wire advance/retract operator not shown in the figures. The treatment unit 42, and the operation tube 12 and operation wire 13, are then coupled together by the same action as in the first embodiment.

When carrying out a procedure using the treatment unit 42, the advance switch 40A is again depressed and the driver 38 is actuated, to advance the operation tube 12 and the operation wire 13.

The operation tube locking part 30 is then pushed toward the front end side by the operation tube 12, so that the spring 45 is compressed, while at the same time the treatment unit 42 is projected out from the front end of the channel 5.

Next, once it has been confirmed using the endoscope 3 that the treatment unit 42 is projecting out by the desired length to reach the lesion site, the advance switch 40A is released, and, with halting of the operation of the driver 38, the movement of the operation tube 12 and the operation wire 13 ceases.

The opening/closing operation of the paired forceps pieces 27 and 28 is carried out by means of the same operation as in the first embodiment.

Once the procedure using the treatment unit 42 is completed, the retract switch 40B is depressed and the driver 38 is actuated to rotate the paired rollers 36 and 37 in the direction opposite the direction of insertion. At this time, the spring 45 begins to expand accompanying the movement of the operation tube 12 and the operation wire 13 toward the base end side of the channel 5.

When the spring 45 recoils to its original length, the spring short tube 46 is released from the advance blocking part 10, and the treatment unit 42 moves within the channel 5 toward the base end side of the inserted part 2 along with the operation tube 12 and the operation wire 13.

Thereafter, the operation tube 12 and the operation wire 13 are wound around the core member 20 by means of the same operation as in the first embodiment. Once the treatment unit 42 has moved to the position of the port 11A, the retract switch 40B is released and the operation of the driver 38 is halted. Next, the concave part 33 and the round member 32 are separated, and the treatment unit 42 is released from the operation tube 12 and the operation wire 13, and recovered.

In this endoscope treatment system 41, the same actions and effects as those of the first embodiment can be obtained. However, in addition, in this endoscope treatment system 41, the spring 45 can be compressed in the axial direction by moving the operation tube 12 and the operation wire 13 further toward the front end of the channel 5 once the operation tube locking part 30 has been locked by the advance blocking part 10 via the spring 45; the treatment unit 42 can be projected outward from the front end of the channel within the limits of the elastic deformation range of the spring 45 by presetting the length of the treatment unit 42 to an optimal length using the sheath 47; and a procedure can be carried out at a site removed from the front end of the inserted part 2 in the same manner as with a regular procedure instrument.

Further, since the paired forceps pieces 27 and 28 are housed inside the spring 45 while being moved inside the channel 5, it is possible to prevent the paired forceps pieces 27 and 28 from opening during movement.

### [Embodiment 3]

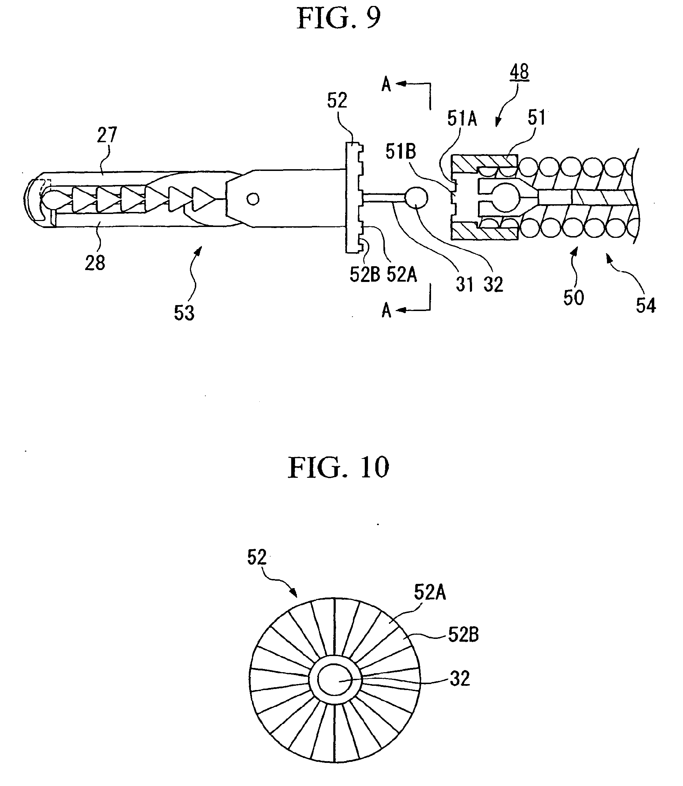

A third embodiment of the present invention will now be explained with reference to FIGS. 9 and 10.

Note that structural elements that are equivalent to those of the preceding embodiments will be assigned the same numeric symbol and an explanation thereof will be omitted here.

The difference between the first and third embodiments is that in the endoscope treatment system 48 according to the third embodiment, tube-side concave parts 51A and tube-side convex parts 51 B are alternately disposed along the circumferential direction of the edge surface of the base 51 at the front end of the operation tube 50, and locking-side convex parts 52A and locking-side concave parts 52B that can engage with the tube-side concave parts 51 A and the tube-side convex parts 51B, respectively, are provided to the end surface of the operation tube locking part 52, as shown in FIG. 10.

The operating method using the endoscope treatment system 48 according to this embodiment consisting of the above design will now be explained below.

First, as in the case of the first embodiment, the treatment unit 53 is inserted into the port 11A, and the operation tube 50 and the operation wire 13 are relayed out from the housing 15 into the channel 5.

When the operation tube 50 and the operation wire 13 are locked by the operation tube locking part 52, the advance switch 40A is released and movement of the operation tube 50 and the operation wire 13 is stopped. The operation wire 13 is rotated about its axis, causing the locking-side convex parts 52A and the locking-side concave parts 52B to engage with the tube-side concave parts 51A and the tube-side convex parts 51B.

Thereafter, the treatment unit 53, and the operation tube 50 and the operation wire 13, are coupled by means of the same operation as in the first embodiment.

By engaging the tube-side concave parts 51 A and the locking-side convex parts 52A, and the tube-side convex parts 51B and the locking-side concave parts 52B in this endoscope treatment system 48, the operation tube 50 and the operation tube locking part 52 are not only locked in the axial direction, but are also locked with respect to rotation about the axis. As a result, the treatment unit 53 and the operation tube 50 and the operation wire 13 can be rotated together, facilitating the rotational operation of the treatment unit 53.

### [Embodiment 4]

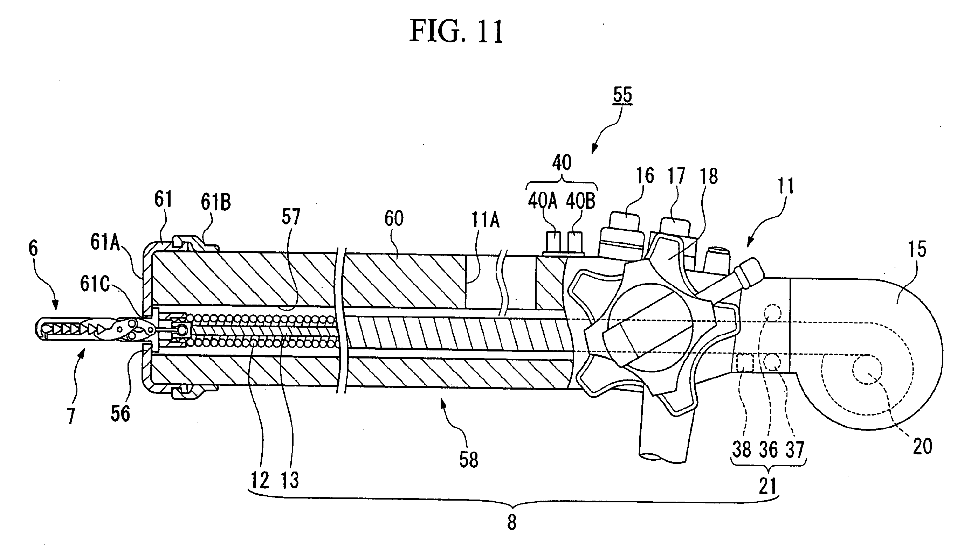

Next, a fourth embodiment of the present invention will be explained with reference to FIG. 11.

Note that structural elements that are equivalent to those of the preceding embodiments will be assigned the same numeric symbol and an explanation thereof will be omitted here.

The point of difference between the fourth and first embodiments is that in the endoscope treatment system 55 according to the fourth embodiment, the advance blocking part 56 is detachably connected to the front end of the channel 57.

This advance blocking part 56 is provided to a cylindrically-shaped front end hood 61 that can be attached to and released from the outer peripheral surface of the front end of the inserted part 60 of the endoscope 58.

An underbody 61A is provided to the front end of the front end hood 61. In addition, an attachment part 61B is disposed to the base end of the front end hood 61 formed to have a profile that covers and engages with the outer peripheral surface of the inserted part 60.

A hole 61C that is smaller than the diameter of the opening of channel 57 is formed in the underbody 61A. Advance blocking part 56 is formed at the peripheral edge of this hole 61C and the edge of the opening of the channel 57.

Note that the underbody 61A avoids the area where the viewing window etc. on the front end surface of the inserted part 60, and is formed in the vertical direction with respect to the axial direction of the front end hood 61.

Next, the operating method using the endoscope treatment system 55 of this embodiment having the design as described above will be explained.

First, the front end hood 61 is attached to the front end of the inserted part 60 prior to inserting the endoscope 58 into the body cavity. The front end hood 61 is attached while adjusting so that the hole 61C and the channel 57 are disposed on the same axis.

Thereafter, as in the first embodiment, the treatment unit 7 is inserted into the port 11A, and the operation tube 12 and the operation wire 13 are relayed out from the housing 15 into the channel 57, and moved toward the front end side, thereby coupling the treatment unit 7 and the operation tube 12 and the operation wire 13.

In this endoscope treatment system 55, the front end locking part 30 can be attached to the channel 57 of a conventional endoscope, and the treatment unit 7 and the operation wire 13 can be coupled inside the channel 57.

Note that various modifications may be made to the preceding embodiments within limits that do not depart from the scope of the invention as defined in the claims.

For example, in the preceding second embodiment, the spring 45 is connected to the operation tube locking part 43 of the treatment unit 42. However, it is also acceptable to connect the front end of the spring 45 to the base end side of the advance blocking part, and to connect the spring short tube to the base end of the spring 45. In addition, the elastic member is not limited to a spring 45, but rather may be an elastic member made of rubber, for example.

In this case as well, the same actions and effects as those of the second embodiment can be obtained.

Further, in the third embodiment, it is acceptable for either the tube-side concave parts or the tube-side convex parts to be disposed to the front end of the operation tube member, and to provide locking-side convex parts or locking-side concave parts to the operation tube locking part that can engage with either the tube-side concave parts or the tube-side convex parts.

### [Embodiment 5]

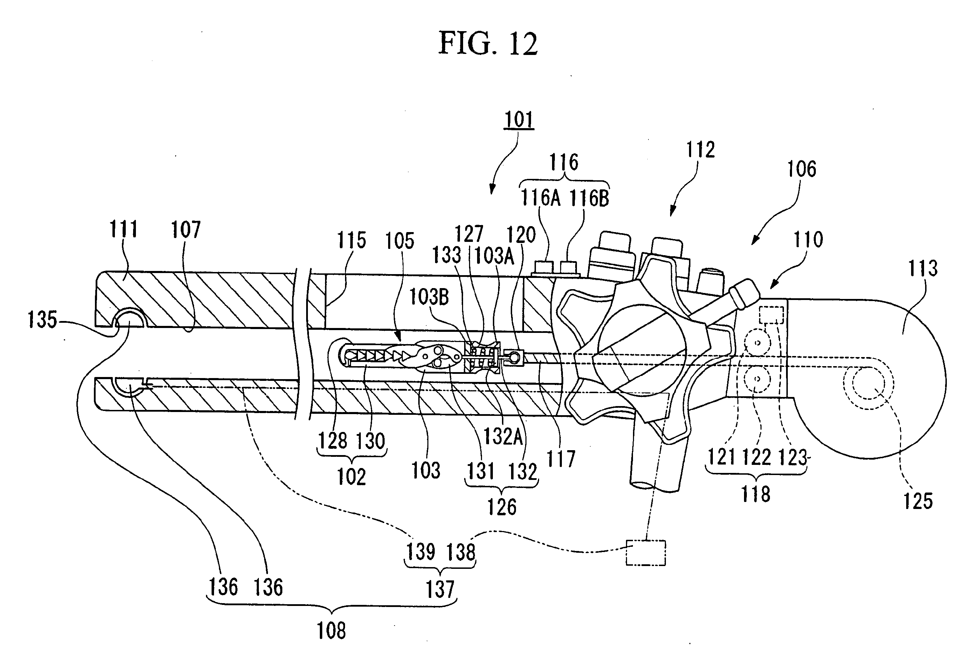

A fifth embodiment of the present invention will now be explained with reference to FIGS. 12 through 14.

The endoscope treatment system 101 according to this embodiment is provided with a treatment unit 105, having a treatment part 102 which receives a driving force to carry out a procedure, and a front end cover (treatment part main body) 103 which supports the treatment part 102; a channel 107 which is formed in the endoscope 106 and into which the treatment unit 105 can be inserted; a restricting means 108 which can restrict the advancing and retracting movement of the treatment unit 105 inside the channel 107; and an advance/retract driving means 110 which advances or retracts the treatment unit 105 between the base end side and the front end side of the channel 107, and which drives the treatment part 102 by advancing or retracting it relative to the front end cover 103 and the channel 107 in a state in which the advance and retraction of the front cover 103 is restricted by the restricting means 108.

Endoscope 106 is provided with a pliable inserted part 111, an operator 112 for operating the inserted part 111, and a housing 113 for housing the advance/retract driving means 110.

A port 115, which communicates along the channel 107 and has an opening that is large enough to enable attachment and release of the treatment unit 105 and the advance/retract driving means 110, and a switch 116 for advancing and retracting the advance/retract driving means 110, are provided to the operator 112.

The switch 116 is provided with an advance switch 116A for advancing the treatment unit 105 toward the front end side of the channel 107, and a retract switch 116B for retracting the treatment unit 105 toward the base end of the channel 107. The advance/retract driving means 110 can be operated by depressing these switches.

The advance/retract driving means 110 is provided with an operation wire (operation line) 117 that is connected to the treatment unit 105 and can advance and retract within the channel 107, and an advance/retract mechanism 118 for advancing and retracting the operation wire 117.

A detachable part 120 is connected to the front end of the operation wire 117 that is capable of connecting or releasing the treatment unit 105.

The advance/retract mechanism 118 is disposed so as to the hold the operation wire 117 under pressure from its side surfaces inside the operator 112, and is provided with a pair of rollers 121 and 122, which can rotate at the same speed in different directions by means of a gear not shown in the figures, and a driver 123, which is connected to roller 121 and rotates and drives this roller.

Housing 113 is connected to the base end side of the operator 112, and is provided with a reel 125. Operation wire 117 can be wound around the outer periphery of this reel 125.

The treatment unit 105 is provided with a treatment part driving mechanism 126 that is supported by the front end cover 103, and is detachably connected to a detachable part 120 provided to the front end of the operation wire 117, wherein the advancing/retracting action of the operation wire 117 with respect to the front cover 103 is communicated to the operator part 102.

The base end 103A side of the front end cover 103 has an underbody 103B that is formed as a cylinder, with a concave-shaped locking part 127 disposed to the outer peripheral surface thereof that is locked by the restricting means 108.

The restricting means 108 is provided imbedded inside the concave part 135 formed in the circumferential direction to the lateral surface of the channel 107 on the front end side of the inserted part 111, and is provided with a convex part 136 that can be recessed in the radial direction of the channel 107 and a recessing mechanism 137 for recessing the convex part 136.

By drawing in air, the convex part 136 is able to expand or contract to a size capable of engaging with the locking part 127 at the front end cover 103. The recessing mechanism 137 is provided with a pump unit 138 for supplying air to the convex part 136 and for drawing out the supplied air, and a vent line 139 that is disposed along the channel 107 and communicates between the pump unit 138 and the convex part 136.

Next, the operating method, the actions and the effects of the endoscope treatment system 101 according to the present embodiment will be explained.

First, the inserted part 111 of an endoscope 106 assembled into the state shown in FIG. 12 is inserted into a body cavity. Next, the advance switch 116A is depressed and the driver 123 is actuated, rotating the paired rollers 121 and 122 in the direction for advancing the operation wire 117 inside the channel 107. As a result, the operation wire 117, held under pressure between the paired rollers 121 and 122, is sent out from the housing 113 into the channel 107.

After moving the front end of the operation wire 117 inside the channel 107 to a position that communicates with the port 115, the advance switch 116A is released, and the driver 123 is temporarily stopped.

The treatment unit 105 is then introduced into the port 115, and the operation wire 117 is attached by connecting a connector 132 and the detachable part 120 of the operation wire 117. After attachment, the advance switch 116A is depressed and the driver 123 is again actuated, moving the operation wire 117 toward the front end side of the channel 107.

Since the link mechanism 131 is in a locked state with respect to the front end cover 103 due to the biasing force of the spring member 133 at this time, the entirety of the treatment unit 105 moves along with the operation wire 117 inside the channel 107 with the paired forceps pieces 128 and 130 in a closed state.

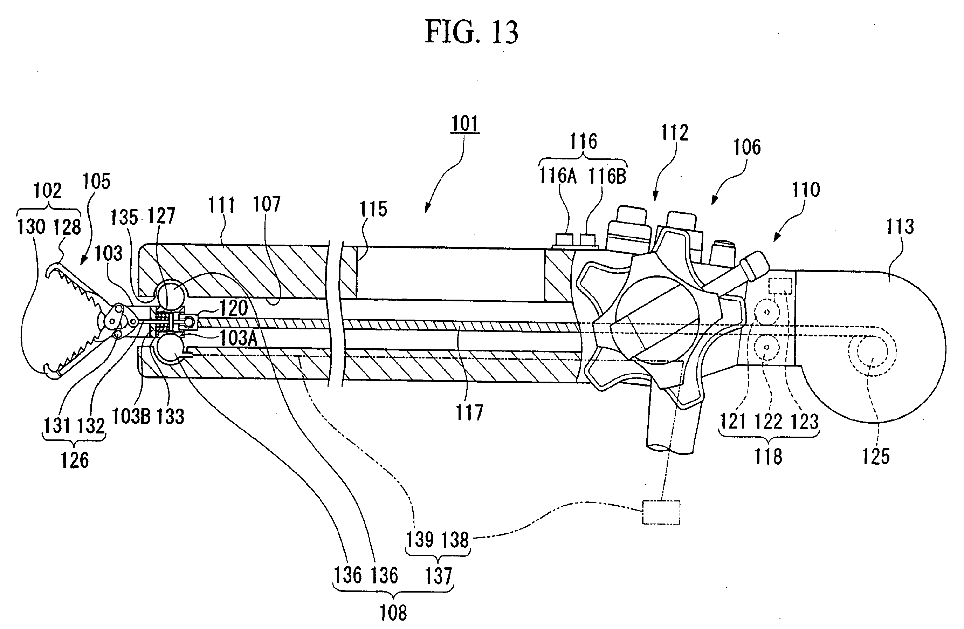

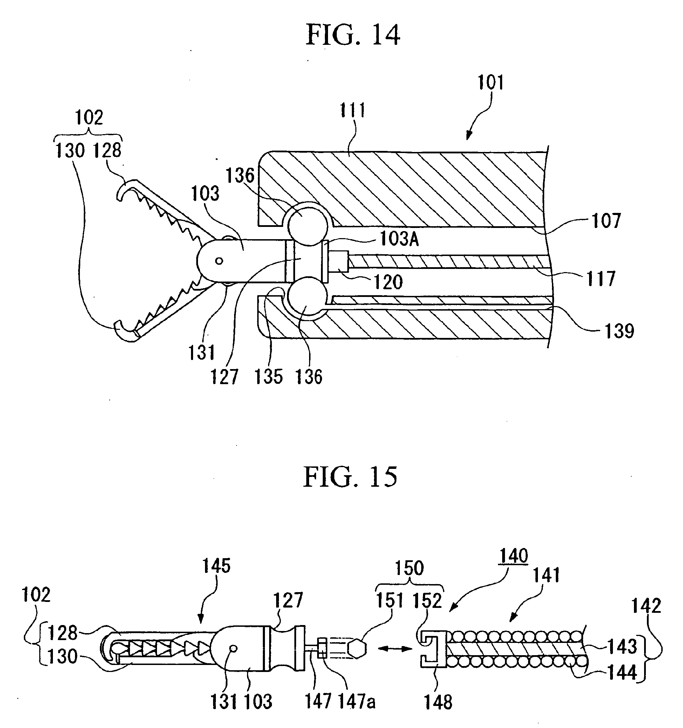

As shown in FIGS. 13 and 14, when the treatment unit 105 is moved to the position of disposition of the convex parts 136, air from the pump unit 138 is sent through the vent line 139 to inflate the convex parts 136. In this case, the convex parts 136 and the locking part 127 are engaged, and the front end cover 103 is engaged inside the channel 107, so that the advance/retract movement of the treatment unit 105 inside the channel 107 is restricted.

The operation wire 117 is moved further toward the front end side of the channel 107 in this state, and is moved forward relative to the front end cover 103 while the spring member 133 is being compressed. As a result, the link mechanism 131 is operated to open the paired forceps pieces 128 and 130.

When the advance switch 116A is released, the rotation of the paired rollers 121 and 122 is stopped and the movement of the operation wire 117 is halted, with the paired forceps pieces 128 and 130 maintained in the open state.

When the paired forceps pieces 128 and 130 are closed, the retract switch 116B is depressed.

The driver 123 is then actuated, causing the paired rollers 121 and 122 to rotate in a direction opposite to that disclosed above, and the operation wire 117 is withdrawn toward the base end side of the channel 107. Since the front end cover 103 is in a locked state in the channel 107 at this time, only the link mechanism 131 is driven, closing the paired forceps pieces 128 and 130.

When the paired forceps pieces 128 and 130 are closed, the pump unit 138 is driven and the air within the convex parts 136 is withdrawn via the vent line 139. As a result, the air within the convex parts 136 is withdrawn, and the convex parts 136 contract into the side surface of the channel 107.

This releases the lock between the front end cover 103 and the channel 107, and the treatment unit 105 is retracted back inside the channel 107 with the paired forceps pieces 128 and 130 closed and biased by the spring member 133.

Once the treatment unit 105 has been retracted back to the port 115, in the case where the next procedure is to be carried out, the treatment unit 105 is exchanged for another treatment unit, and the operations described above are repeated.

In this endoscope treatment system 101, the treatment unit 105 is inserted into the channel 107 via the port 115, and is connected to the operation wire 117. As a result, the operation wire 117 can be advanced or retracted within the channel using the advance/retract mechanism 118. In addition, by moving the treatment unit 105 to the front end of the channel 107, and causing the convex part 136 to project out into the channel 107 using the recessing mechanism 137, it is possible to stop the advance/retract movement of the treatment unit 105 inside the channel 107. In addition, driving of the treatment part 102 can be carried out by further advancing or retracting the operation wire 117.

Since the spring member 133 biases the link mechanism 131 in the direction that closes the paired forceps pieces 128 and 130 at this time, it is possible to prevent the accidental opening of the paired forceps pieces 128 and 130 as the treatment unit 105 is being advanced or retracted inside the channel 107.

In addition, when carrying out a plurality of different procedures, it is not necessary to exchange the entire procedure instrument as in the case of the conventional art, but rather it is possible to respond to a variety of procedures by exchanging only the treatment unit 105.

### [Embodiment 6]

Next, a sixth embodiment of the present invention will now be explained with reference to FIG 15.

Note that structural elements that are equivalent to those of the fifth embodiment will be assigned the same numeric symbol and an explanation thereof will be omitted here.

The point of difference between the sixth and fifth embodiments is that the operation wire 142 of the endoscope 141 in the endoscope treatment system 140 according to the sixth embodiment is provided with a wire main body 143 and a winding coil (helical member) 144 covering this wire main body 143.

In addition, a locking mechanism 150 is provided for enabling rotation about the center axis of the connector part 147 of the treatment part driving mechanism in the treatment unit 145 and the detachable part 148 of the operation wire 142 which are in a mutually locked state.

The detachable part 148 is connected to the front end of the wire main body 143 and the winding coil 144.

The locking mechanism 150 is provided with a corner part 151 which is formed to the outer peripheral surface of the end 147a of the connector 147, and a receiving part which can engage with the end 147a inside detachable part 148. By engaging this corner part 151 and the receiving part 152, the rotation of the treatment unit 145 and the operation wire 142 around their respective center axes is restricted, and the treatment unit 145 and the operation wire 142 can rotate together.

The same actions and effects can be obtained with this endoscope treatment system using the same operating method described above.

In this case, even if a load in the compressing direction is applied to the operation wire 142 in the state where the front end cover 103 is locked in the channel 107, the buckling strength of the operation wire 142 is increased due to the winding coil 144, so that buckling of the operation wire 142 can be prevented, and the opening/closing operation of the paired forceps pieces 128 and 130 can be carried out smoothly. In addition, even if traction force is applied on the operation wire 142, the amount of extension of the operation wire 142 can be controlled due to the winding coil 144, so that an accurate operation can be carried out at the desired time.

In addition, when the treatment unit 145 is rotated around its center axis during a procedure, it is possible to transmit the rotational force to the treatment unit 145 via the engaging mechanism 151 by rotating the operation wire around its center axis. As a result, the procedure can be carried out more accurately and easily.

### [Embodiment 7]

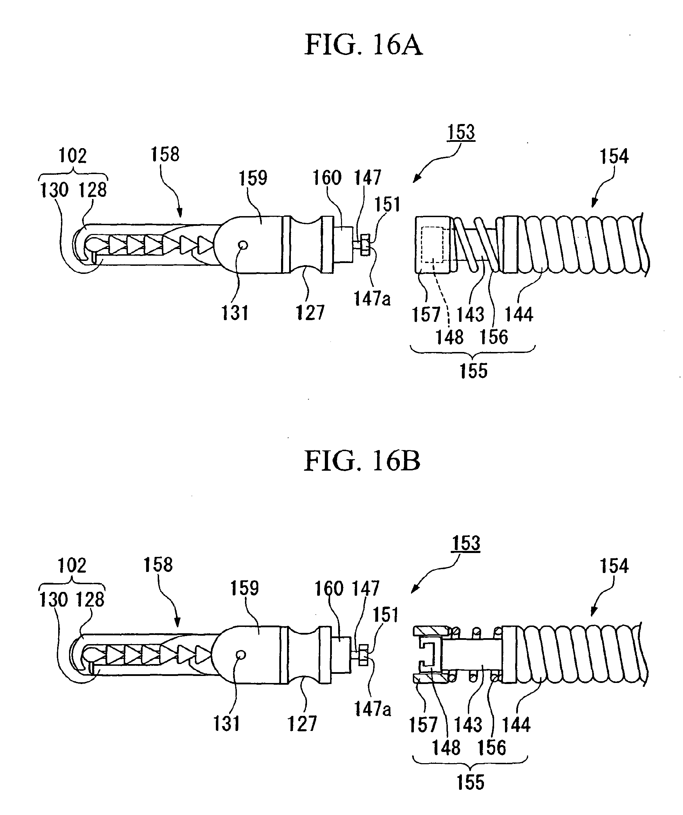

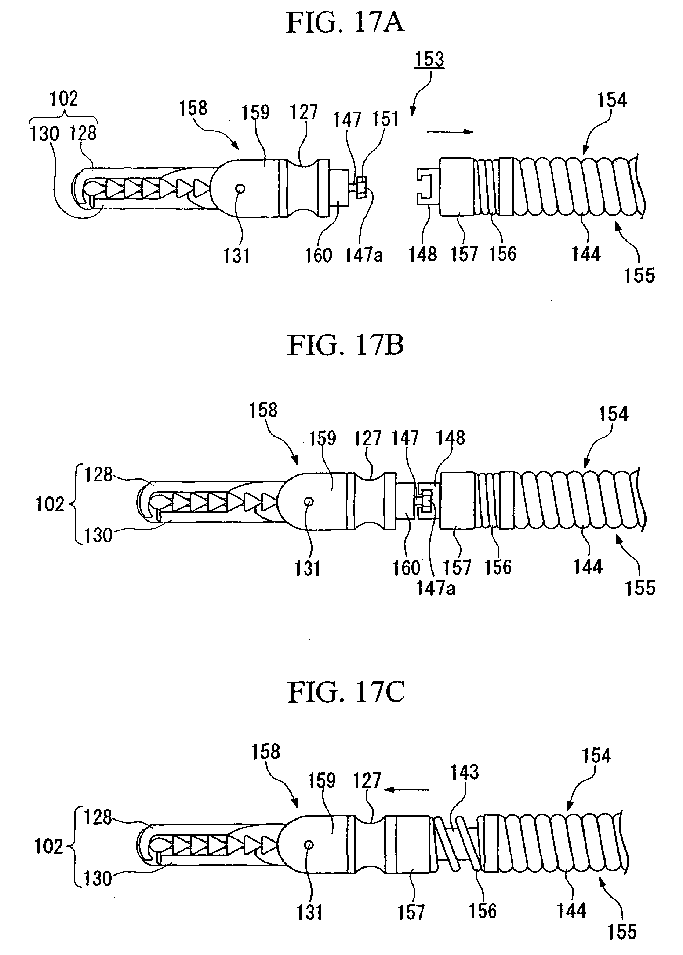

Next, a seventh embodiment of the present invention will now be explained with reference to FIGS. 16 and 17.

Note that structural elements that are equivalent to those of the above embodiments will be assigned the same numeric symbol and an explanation thereof will be omitted here.

The point of difference between the seventh and sixth embodiments is that the detachable part 148 and the front end of the winding coil 144 of the operation wire 155 in the advance/retract driving means 154 of the endoscope treatment system 153 according to the seventh embodiment are connected mutually via a spring 156 as shown in FIGS. 16A and 16B.

The link member 157 having an outer diameter roughly the same as the winding coil 144 for covering the detachable part 148 is connected to the front end of the spring 156.

A convex engaging part 160 capable of engaging with the inside of the link member 157 is disposed to the base end of the front end cover 159 of the treatment unit 158.

Next, the operating method, actions and effects of the endoscope treatment system 153 according to the present embodiment.

First, as in the case of the fifth embodiment, the inserted part 111 of the endoscope 106 is inserted into the abdominal cavity. The advance switch 116A is depressed and the driver 123 is actuated, the paired rollers 121 and 122 are rotated in the direction that advances the operation wire 155 inside the channel 107, and the operation wire 155 is sent out from the housing 113 to the channel 107.

When the front end of the operation wire 155 is moved to the port 115, the movement of the operation wire 155 is stopped, and the treatment unit 158 is introduced into the port 115, and is connected to the operation wire 155.

As shown in FIG. 17A, the detachable part 148 is exposed by moving the link member 157 to the base end side of the operation wire 155 while compressing the spring 156. Next, as shown in FIG. 17B, the end 147a of the connector 147 of the treatment unit 158 and the detachable part 148 of operation wire 155 are connected.

When the force gripping the link member 157 is lessened in this state, the link member 157 moves toward the treatment unit 158 side due to the elastic force of the spring 156, and as shown in FIG. 17C, the link member 157 and the engaging part 160 are engaged.

In this way, the operation wire 155 and the treatment unit 158 are connected. After connection, the treatment unit 158 is advanced or retracted within the channel 107 by advancing or retracting the operation wire 155, to carry out the opening/closing operation of the paired forceps pieces 128 and 130.

In this endoscope treatment system 153, it is possible to more strongly connect the treatment unit 158 and the operation wire 155, so that they do not separate within the channel 107.

In addition, it is possible pull the operation wire 155 toward the base end by means of the elastic force of the spring 156, and bias the paired forceps pieces 128 and 130 closed.

### [Embodiment 8]

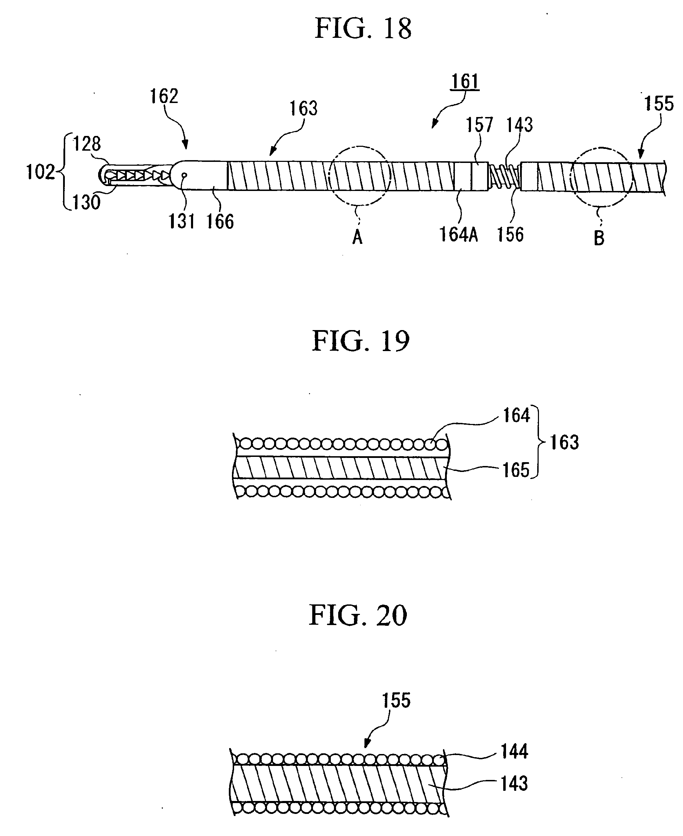

Next, an eighth embodiment of the present invention will be explained with reference to FIGS. 18 to 21.

Note that structural elements that are equivalent to those of the above embodiments will be assigned the same numeric symbol and an explanation thereof will be omitted here.

The point of difference between the eighth embodiment and the seventh embodiment is that the endoscope treatment system 161 according to the eighth embodiment is provided with an extension part 163 that is able to come into contact with the convex part 136, is connected to the treatment unit 162 and the operation wire 155, and is formed to have a length capable of projecting the treatment unit 162 out from the front end of the channel 107.

As shown in FIGS. 18 through 20, the extension part 163 is provided with a sheath tubing 164 that is formed in a coil-shape having a specific length, and a wire part 165 disposed so that it can advance and retract inside the sheath tubing 164 with respect to the sheath tubing 164.

The outer diameter of the sheath tubing 164 is formed to be a size such that it presses into contact with the convex part 136 during expansion of the convex part 136, and the front end thereof is connected to the base end of the front end cover 166. In addition, a cylindrical part 164A that can engage with the linking member 157 is connected to the base end of the sheath tubing 164.

The front end of the wire part 165 is connected to the base end of the linking mechanism 131, and the base end of the wire part 165 can be joined with the operation wire 155.

Next, the operating method, actions and effects of the endoscope treatment system 161 according to this embodiment will be explained.

First, as in the case of the fifth embodiment, the inserted part 111 of the endoscope 106 is inserted into a body cavity. The advance switch 116A is depressed, actuating the driver 123 and causing the paired rollers 121 and 122 to rotate in the direction that advances the operation wire 155 inside the channel 107. As a result, the operation wire 155 is sent out from the housing 113 into the channel 107.

When the front end of the operation wire 155 is moved to the port 115, the treatment unit 162 is inserted from the port 115 into the channel 107. Using the same method as employed in the seventh embodiment, the operation wire 165 and the wire main body 143, and the cylindrical part 164A and the linking member 157, are respectively connected, attaching the extension part 163 to the operation wire 155.

After attachment, the advance switch 116A is depressed, again actuating the driver 123, and the operation wire 155 is moved toward the front end side of the channel 107 in the same manner as in the fifth embodiment.

The movement of the operation wire 155 is then stopped with the treatment part 102 projected out a specific distance from the front end of the channel 107.

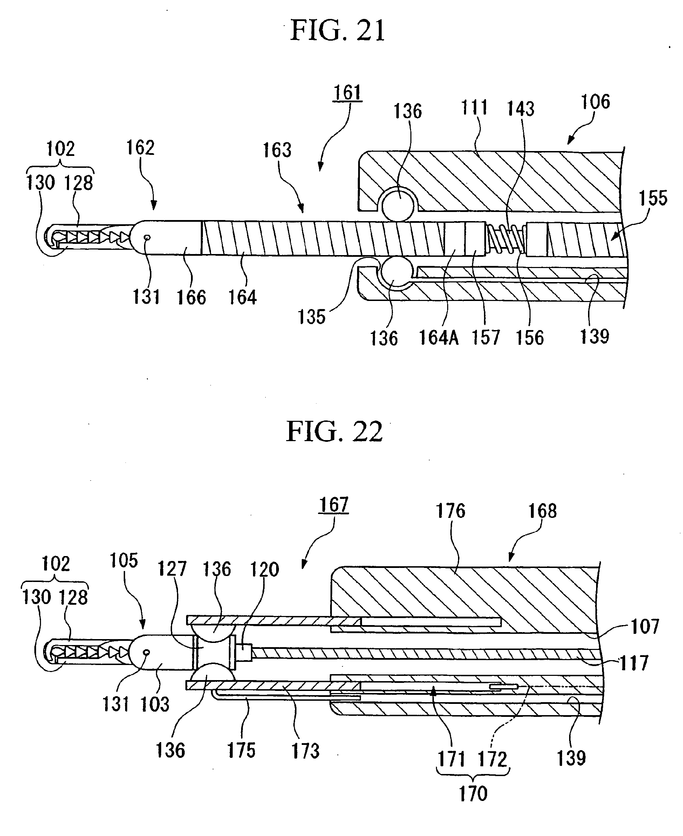

Next, air is relayed through the vent line 139 from the pump unit 138 to inflate the convex part 136 in the same manner as in the fifth embodiment. As a result, the convex part 136 and the sheath tubing 164 engage as shown in FIG. 21, so that the extension part 163 is locked within the channel 107, and movement of the treatment unit 162 and the extension part 163 with respect to the channel 107 is restricted.

When the operation wire 155 is moved to the front end side of the channel 107 in this state, the wire part 165 which is connected to the operation wire 155 advances relative to the sheath tubing 164 and the front end cover 166 which is connected thereto, manipulating the link mechanism 131 and opening the paired forceps pieces 128 and 130.

Next, the advance switch 116A is released, the rotation of the paired rollers 121 and 122 is stopped, and the movement of the operation wire 155 is halted.

When the paired forceps pieces 128 and 130 are closed, the retract switch 116B is depressed, and the operation wire 155 is retracted toward the base end side of the channel 107 in the same manner as in the fifth embodiment. The front end cover 166 and the sheath tubing 164 are halted with respect to the channel 107 at this time. The wire member 165 is advanced with respect to the front end cover 166, and the link mechanism 131 is activated, closing the paired forceps pieces 128 and 130.

When the paired forceps pieces 128 and 130 are closed, the pump unit 138 is activated and the convex part 136 is contracted into the lateral surface of the channel 107. As a result, the state of engagement between the extension part 163 and the channel 107 is released and the extension part 163 becomes free with respect to the channel 107.

The treatment unit 162 is then retracted within the channel 107.

In this endoscope treatment system 161, it is possible to carry out both movement of the treatment unit 162 inside the channel 107 and the opening/closing operation of the paired forceps 128 and 130 by attaching an extension part 163 of the desired length to the operation wire 155, and then advancing or retracting this operation wire 155. In addition, the treatment unit 162 can be made to project out from the front end of the channel 107 by a desires amount, so that a procedure can be carried out on a lesion site that is located at a site removed from the front end of the channel 107.

### [Embodiment 9]

Next, a ninth embodiment of the present invention will be explained with reference to FIG 22.

Note that structural elements that are equivalent to those of the preceding embodiments will be assigned the same numeric symbol and an explanation thereof will be omitted here.

The difference between the ninth and eighth embodiments is that an extension part 163 is provided linking the area between the treatment unit 162 and the operation wire 155 in the eighth embodiment, while a projecting mechanism 170 capable of causing the convex part 136, along with a part of the channel 107, to project out from the front end of the channel 107 is provided to the endoscope 168 of the endoscope treatment system according to the ninth embodiment.

The projecting mechanism 170 is provided with a pneumatic cylinder 171 that is disposed to the front end of the channel 107, and an airline 172 that is connected to the pump unit 138 for relaying gas to the pneumatic cylinder 171 separate from the relay of gas to the convex part 136.

The convex part 136 is disposed to the front end of the cylinder 173 of the pneumatic cylinder 171, and the front end 175 of the vent line 139 can be moved along with the movement of the pneumatic cylinder 171.

Next, the operating method, actions and effects of the endoscope treatment system 167 according to this embodiment will be explained.

First, as in the fifth embodiment, the inserted part 176 of the endoscope 168 is inserted into the body cavity. The advance switch 116A is depressed and the driver 123 is actuated, so that the paired rollers 121 and 122 are rotated in the direction that advances the operation wire 117 inside the channel 107. As a result, the operation wire 117 is relayed out from the housing 113 into the channel 107, and the treatment unit 105 is attached to the operation wire 117 by the same method as that in the fifth embodiment.

The operation wire 117 is again moved toward the front end side of the channel 107. Once it reaches the treatment unit 105 reaches the position of the convex part 136, air is relayed from the pump unit 138 to the air vent 139, to inflate the convex part 136. As aresult, the convex part 136 and the locking part 127 engage, and the front end cover 103 is locked inside the channel 107, so that advancing and retracting movement of the treatment unit 105 inside the channel 107 is restricted.

Air is then supplied under this condition to the air line 172, to drive the pneumatic cylinder 171.

The treatment unit 105 projects out from the front end of the channel 107 along with the convex part 136, accompanying the projection of the cylinder 173 from the front end of the channel 107.

After projecting the treatment unit 105 out a specific length, the operation wire 117 is moved further toward the front end of the channel 107, advancing relative to the front end cover 103. As a result, the spring 156 is compressed, operating the link mechanism 131 and opening the paired forceps pieces 128 and 130.

When the advance switch 116A is released, the rotation of the paired rollers 121 and 122 is stopped and the movement of the operation wire 117 is halted, with the paired forceps pieces maintained in the open state.

The retract switch 116B is depressed and the paired forceps pieces 128 and 130 are closed, after which the pneumatic cylinder 171 is withdrawn back to the initial position. The operation wire 117 is withdrawn, and the cylinder part 173, convex part 136 and the treatment unit 105 are moved together inside the channel 107, after which the convex part 136 is further contracted, so that the treatment unit 105 becomes free with respect to the channel 107, and the treatment unit 105 is recessed into the channel 107.

By driving the projecting mechanism 170 in this endoscope treatment system 167, the treatment unit 105 can be projected out from the front end of the channel 107 by the desired amount. As a result, as in the eighth embodiment, a procedure can be carried out to a lesion site that is separated from the front end of the channel 107.

### [Embodiment 10]

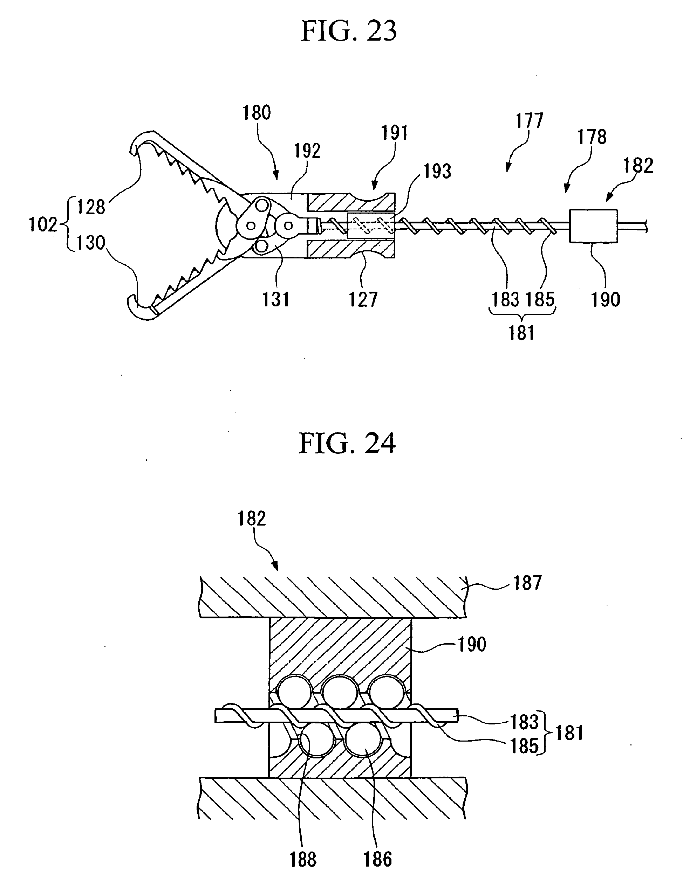

Next, a tenth embodiment of the present invention will be explained with reference to FIGS. 23 and 24.

Note that structural elements that are equivalent to those of the preceding embodiments will be assigned the same numeric symbol and an explanation thereof will be omitted here.

The difference between this embodiment and the tenth embodiment is that the advance/retract driving means 178 of the endoscope treatment system 177 according to this embodiment is capable of advancing and retracting inside the channel 107 by being supplied with a rotational force from a motor (rotation driving means) not shown in the figures, for example, and is attached to the treatment unit 180 to drive it.

As shown in FIG. 23, the advance/retract driving means 178 is provided with an operation wire 181; a roller not shown in the figures that is connected to the motor and is for rotating the operation wire 181; and a rotation mechanism 182 for converting the rotation action of the operation wire 181 into advancing/retracting motion inside the channel 107.

The operation wire 181 is provided with a wire main body 183, and a helical part 185 that is wrapped in a helical shape around the outer peripheral surface of the wire main body 183.

As shown in FIG. 24, the rotation mechanism 182 is provided with a plurality of balls 186 disposed in array to enable rotation between the operation wire 181 and the helical part 185; and a mechanism main body 190 that is fixed in place to the endoscope 187 and in which a helical groove 188 is formed through which the balls 186 can move.

The treatment part driving mechanism 191 in the treatment unit 180 is provided with a converting mechanism 193 having the same structure as the rotation mechanism 182 and is connected to the base end side of the front end cover 192, with the base end of the link mechanism 131 and the front end of the operation wire 181 directly connected.

A locking part 127 for restricting rotation with respect to the channel 107 by engaging the convex part 136 is formed to the front end cover 192.

Next, the operating method, actions and effects of the endoscope treatment system 177 according to this embodiment will be explained.

First, the inserted part 111 of the endoscope 187 is inserted into a body cavity. The advance switch 116A is depressed and a motor, not shown in the figures, rotates the operation wire 181 about its center axis.

The balls 186 turn inside the helical groove 188 in the helical part 185 of the wire main body 183, and the channel 107 advances while the operation wire 181 is rotated around its center axis by the rotation mechanism 182.

When the treatment unit 180 is moved to the position of the convex part 136, the convex part 136 is inflated in the same manner as in the fifth embodiment, causing the convex part 136 and the locking part 127 to engage, so that the front end cover 192 is locked inside the channel 107.

The operation wire 181 is then further rotated in this state. Since the converting mechanism 193 does not rotate with respect to the rotation of the operation wire 181, the operation wire 181 is advanced relative to the front end cover 192 by the same action as the rotation mechanism 182. The link mechanism 131 is thus manipulated to open the paired forceps pieces 128 and 130.

Next, the retract switch 116B is depressed and the motor is rotated in the opposite direction from advance.

As the operation wire 181 is rotated with respect to the front end cover 192 in the direction opposite that above, it retracts back toward the base end side of the channel 107, closing the paired forceps pieces 128 and 130. Next, when the convex part 136 is contracted, the treatment unit 180 becomes free with respect to the channel 107. By further retracting the operation wire 181, the treatment unit 180 is recessed into the channel 107.

In this endoscope treatment system 177, by rotating the operation wire 181, it is possible to retract the treatment unit 180 inside the channel 107 via the rotation mechanism 182, and to carry out the opening/closing operation of the paired forceps pieces 128 and 130 via the converting mechanism 193.

### [Embodiment 11]

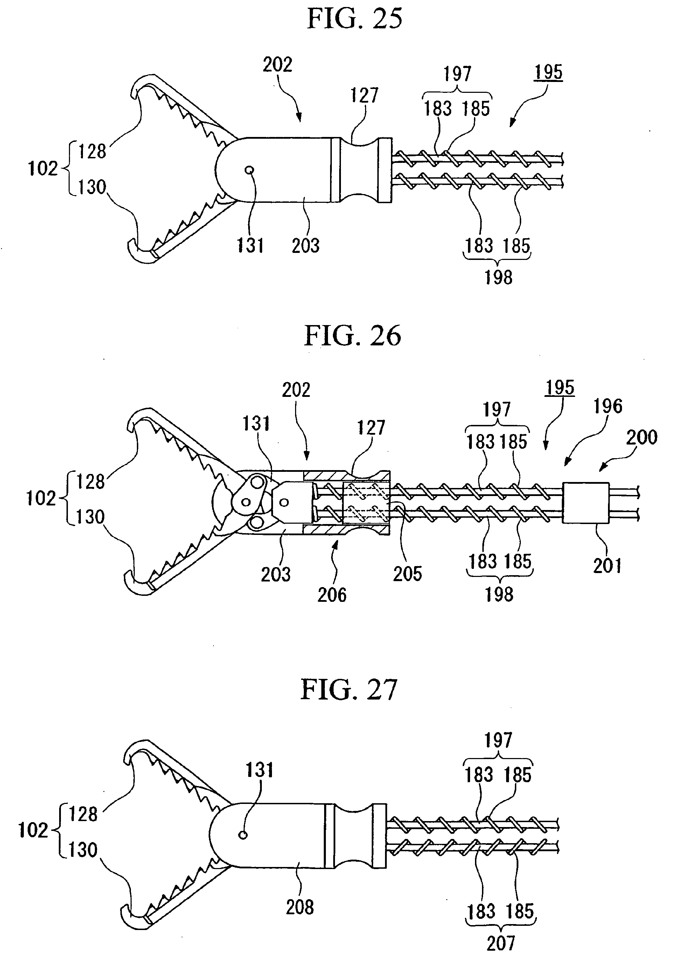

Next, an eleventh embodiment of the present invention will be explained with reference to FIGS. 25 through 27.

Note that structural elements that are equivalent to those of the preceding embodiments will be assigned the same numeric symbol and an explanation thereof will be omitted here.

The difference between the eleventh embodiment and the tenth embodiment is that the advance/retract driving means 196 of the endoscope treatment system 195 according to this embodiment is provided with a first operation wire 197 and a second operation wire 198.

Referring to FIGS. 25 and 26, the mechanism main body 201 of the rotation mechanism 201 has the same design as the tenth embodiment with respect to the first operation wire (operation line) 197 and the second operation wire (operation line) 198.

The treatment unit 202 is provided with a front end cover 203 in which the locking part 127 is formed, and a treatment part driving mechanism 206 consisting of a converting mechanism 205 having the same structure as the mechanism main body 201.

In this endoscope treatment system 195, by rotating the first operation wire 197 and the second operation wire 198 simultaneously in the same direction and at the same speed, the treatment unit 202 can be advanced in the same manner as in the tenth embodiment, to obtain the same effects as described above. In this case, unlike the tenth embodiment, the rotational torque generated in the front end cover 203 by the rotation of one of either the first operation wire 197 or the second operation wire 198 can be restricted by the rotation of the other wire, so that advancing and retracting can be carried out under conditions such that the front end cover 203 does not rotate. Accordingly, it is acceptable that the convex part 136 restrict only the movement in the advance/retract direction of the front cover 203.

In addition, as shown in FIG. 27, it is acceptable that the orientation of the helical part 185 of the second operation wire 207 be in a direction different than that of the first operation wire 197. As a result, it is possible to further reduce the rotational torque of the front end cover 208.

### [Embodiment 12]

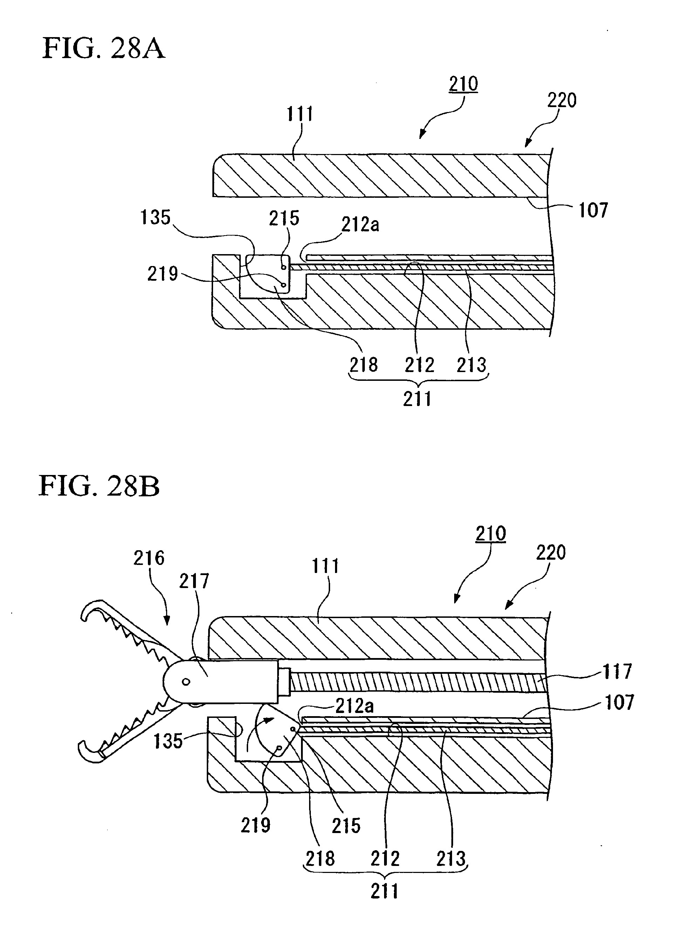

Next, a twelfth embodiment of the present invention will now be explained with reference to FIG. 28.

Note that structural elements that are equivalent to those of the preceding embodiments will be assigned the same numeric symbol and an explanation thereof will be omitted here.

The difference between the twelfth embodiment and the other embodiments described above is that in the preceding embodiments the restricting means 108 is provided with a convex part 136, and a recessing mechanism 137 for recessing the convex part 136; the convex part 136 can be extended or contracted to a size capable of engaging with the locking part 127 on the front end cover 103 by drawing air internally; and the recessing mechanism 137 is provided with a pump unit 138 and a vent line 139. However, in the endoscope treatment system 210 according to this embodiment, the restricting means 211 is provided with an operating axis member 213 which is disposed extending along the channel 107 inside a path 212, and a presser plate 218 that is disposed inside the concave part 135 and is connected via a first rotation axis 215 that is disposed to enable rotation at the front end of the operating axis member 213, the presser plate 218 being capable of engaging with the front end cover 217 of the treatment unit 216.

As shown in FIG. 28A, the presser plate 218 is pinnate in form, and the first rotation axis 215 is disposed to the position of the pinnate-shaped main part. One end of the presser plate 218 is connected to enable rotation to a second rotation axis 219 that is disposed inside the concave part 135.

The operating method, actions and effects of this endoscope treatment system 210 will now be explained.

First, the inserted part 111 of the endoscope 220 is inserted into a body cavity, and the treatment unit 216 is moved to a position on the front end side of the channel 107 along with the operation wire 117 in the same manner as in the fifth embodiment.

When opening the paired forceps pieces 128 and 130, the operating axis member 213 is moved toward the base end side of the channel 107.

As shown in FIG. 28B, a rotational torque is applied to the presser plate 218 about the second rotation axis 219 via the first rotation axis 215. Accordingly, the presser plate 218 rotates about the second rotation axis 219 until the first rotation axis 215 comes into contact with the end 212a of passage 212 that is open to the concave part 135 of the path 212.

Accompanying this rotation, the other end of the presser plate 218 comes into contact with the treatment unit 216, and the front end cover 217 is pushed against the side surface of the channel 107, so that the front end cover 217 is locked in the channel 107.

Thereafter, the opening/closing operation of the paired forceps pieces 128 and 130 is carried out in the same manner as in the fifth embodiment.

After the paired forceps pieces 128 and 130 are closed, the operating axis member 213 is pushed out toward the front end side of the channel 107.

The presser plate 218 separates from the open end 212a, and rotates in a direction opposite that disclosed above about the rotation axis 215 while moving inside the concave part 135. As a result, the treatment unit 216 separates from the side surface of the channel 107. Next, the treatment unit 216 moves toward the base end side of the channel 107 along with the operation wire 117.

Using this endoscope treatment system, the same effects can be obtained as in the fifth embodiment.

Note that various modifications may be made to the preceding embodiments provided that they do not depart from the scope of the present invention.

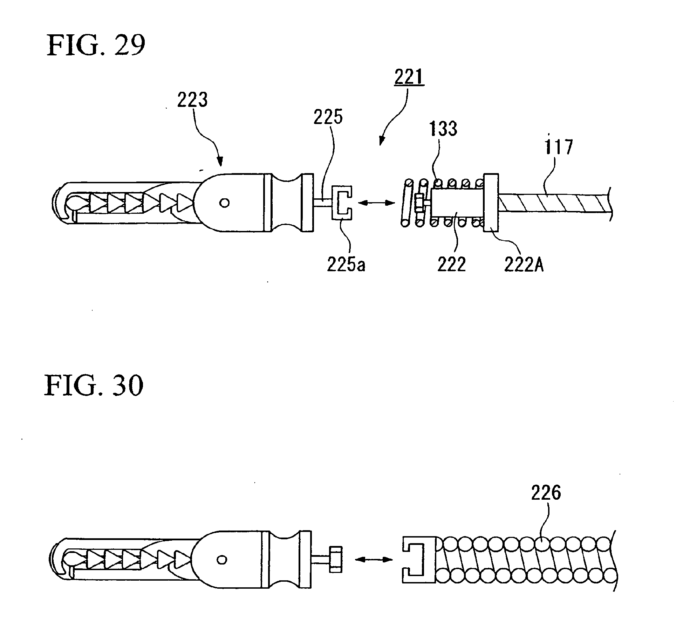

For example, in the preceding embodiments, a spring member 133 is provided to the connector 132. However, as shown in FIG. 29, it is also acceptable that the detachable part 222 in the endoscope treatment system 221 be connected to the operation wire 117 via a flange 222A that is of a larger diameter than the spring member 133, and the this spring member 133 be disposed to the detachable part 222.

In this case, when connecting the tube part 225a of the connector 225 of the treatment unit 223 to the detachable part 222 of the operation wire 117, the spring member 133 is compressed, so that there is biasing in a direction that moves the connector 218 toward the base end side of the channel, enabling biasing to close the paired forceps pieces 128 and 130.

In addition, as shown in FIG. 30, an operation tube 226 wound in the form of a coil can be employed in place of the operation wire 117. In this case, operation tube 226 can provide greater rigidity with respect to either buckling or traction than the operation wire 117, and the operation of opening and closing the paired forceps pieces 128 and 130 can be carried out greater certainty.

In addition, in the preceding tenth embodiment, the rotated operation wire 181 is retracted within the channel 107 by the rotation mechanism 182. However, in contrast to this, it is also acceptable to provide an advance/retract mechanism 118 similar to tat of the fifth embodiment in place of the rotation mechanism 182, and carry out the advance and retract operation of the operation wire 181 with the advance/retract mechanism 118.

In this case, after using the advance/retract mechanism 118 to move the treatment unit 180 to the front end side of the channel 107, the operation wire 181 can be rotated in the same manner as in the tenth embodiment, enabling opening and closing of the paired forceps pieces 128 and 130. Accordingly, it is acceptable to provide only a wire main body 183 to the base end side of the operation wire 181, and not dispose a helical part 185.

In addition, in the above case, since it is acceptable to restrict only the rotation of the treatment unit 180 during opening and closing of the paired forceps pieces 128 and 130, a flat surface part may be provided at which the front end part of the channel and the front end cover can mutually engage, in place of the convex part 136, and restrict the rotation of these parts in this manner.

In the preceding eleventh embodiment, the first procedure wire 197 and the second procedure wire 198 are made to rotate, and this rotational force is converted into an advance/retract driving force by rotation mechanism 200, to advance or retract the treatment unit 202. However, as above, it is acceptable to proved an advance/retract mechanism in place of the rotation mechanism 200 that can advance or retract the first operation wire 197 and the second operation wire 198 at the same speed simultaneously along the channel 107.

In this case as well, the opening/closing operation of the paired forceps pieces 128 and 130 can be carried out in a state such that the rotational torque generated in the front end cover 203 by the rotation of either the first operation wire 197 or the second operation wire 198 is limited by the rotation of the other wire.

Preferred embodiments of the present invention were described above, however, the present invention is not limited thereto. Rather, various structural additions, omission, substitutions and other alterations are possible, provided that they do not depart from the scope of the invention. Moreover, the present invention is not limited by the preceding discussion, but rather, is limited only by the appended claims.

### INDUSTRIAL APPLICABILITY

According to the present invention, the endoscope treatment system for inserting the treatment unit with the channel enables the treatment unit, and the operation tube and operation line, to be connected by means of a unidirectional continuous operation, and makes it possible to manipulate the treatment unit, to carry out a procedure in shorter period of time, more easily.

Moreover, with the endoscope treatment system according to the present invention, the treatment part can be manipulated even without providing to the end of a procedure instrument an operator for manipulating the treatment part, as has been required conventionally. Accordingly, the technician can carry out the procedure using the procedure instrument while manipulating the endoscope, and the time required for the procedure can be reduced. In addition, since a coil sheath such as employed in the conventional art is not required for the procedure instrument, a more compact housing can be accomplished.

## Claims

1. An endoscope treatment system (4, 41, 48, 55, 101, 140, 153, 161, 167, 177, 195, 210, 221) comprising:
an endoscope (3, 106, 141, 168, 187) having an inserted part (2, 111, 176);
a channel (5, 57, 107) provided along said inserted part (2, 111, 176);
a treatment unit (7, 105, 145, 158, 162, 180, 202, 216, 223) which can be inserted into said channel (5, 57, 107) and has a treatment part (6, 102) at its front end; and
a treatment unit driving mechanism (8, 110, 126, 154, 158, 178, 196) for moving said treatment unit (7, 105, 145, 158, 162, 180, 202, 216, 223) inside said channel (5, 57, 107);
**characterized by** further comprising a locking member (10, 56, 108, 127, 211) provided on a side of a front end of the channel (5, 57, 107) in said inserted part (2, 111, 176), and adapted for restricting a projection amount of the treatment unit (7, 105, 145, 158, 162, 180, 202, 216, 223) from the front end of the channel (5, 57, 107) by locking said treatment unit (7, 105, 145, 158, 162, 180, 202, 216, 223) when said treatment unit (7, 105, 145, 158, 162, 180, 202, 216, 223) is being moved forward.

2. An endoscope treatment system according to claim 1, **characterized in that**:
said treatment unit driving mechanism (8, 110, 126, 154, 158, 178, 196) is an advance driving mechanism for advancing said treatment unit (7, 105, 145, 158, 162, 180, 202, 216, 223) within said channel (5, 57, 107) toward the front end of said channel (5, 57, 107).

3. An endoscope treatment system according to claim 2, **characterized in that** said treatment unit (7, 105, 145, 158, 162, 180, 202, 216, 223) is provided with a treatment part driving mechanism (25, 126, 191, 206) for receiving the advance/retract driving force and driving said treatment part (6, 102), and a connecting mechanism (26, 132, 147, 225) for connecting the front end of said advance driving mechanism (8, 110, 118, 154, 178, 196) and the base end side of said treatment part driving mechanism (25, 126, 191, 206), by advancing said advance driving mechanism (8, 110, 126, 154, 158, 178, 196) under the condition where said treatment unit (7, 105, 145, 158, 162, 180, 202, 216, 223) is locked by said locking member (10, 56, 108, 127).

4. An endoscope treatment system according to claim 3, **characterized in that**:
said advance driving mechanism (8, 110, 126, 154, 158, 178, 196) is provided with a tubular operation tube (12, 50), and an operation line (13, 117, 142, 155, 181, 197, 198) that is disposed to enable advance and retraction along the axial direction of said operation tube (12, 50) and is for connecting to said treatment part driving mechanism (25, 126, 191, 206) by said connecting mechanism (26, 132, 147, 225) ; and
said treatment unit (7, 105, 145, 158, 162, 180, 202, 216, 223) is provided with an operation tube locking part (30, 52) for locking said operation tube (12, 50).

5. An endoscope treatment system according to claim 4, **characterized in that** said connecting mechanism (26, 132, 147, 225) is provided with:
a coupler main body (31) formed in the shape of a rod projecting out from the base end side of said treatment unit (7, 105, 145, 158, 162, 180, 202, 216, 223);
a round member (32) disposed to the side edge of said operation tube (12, 50) of said coupler main body (31) and having a diameter that is larger than the outer diameter of said coupler main body (31);
a concave part (33) that is disposed to the front end of said operation line (13, 117, 142, 155, 181, 197, 198) and is for engaging with said round member (32); and
a slit (35) for guiding said round member (32) into said concave part (33), in which the width of the opening is smaller than the diameter of said round member (32) and which can be elastically deformed to enable insertion of said round member (32).

6. An endoscope treatment system according to claim 2, **characterized in that** said locking member(10, 56, 108, 127) is detachable with respect the front end of said channel (5, 57, 107).

7. An endoscope treatment system according to claim 4, **characterized in that** an elastic member (45) that can freely expand and contract in the axial direction is disposed between said operation tube locking part (30, 52) and said locking member (10, 56, 108, 127, 211).

8. An endoscope treatment system according to claim 4, **characterized in that**:
at least one of either a tube-side concave part (51A) or a tube-side convex part (51B) is disposed to the front end of said operation tube (12, 50); and
at least one of either a locking-side convex part (52A) or a locking-side concave part (52B) that can engage with at least one of either the tube-side concave part (51A) or the tube-side convex part (51B) is disposed to said operation tube locking part (30, 52).

9. An endoscope treatment system according to claim 1, **characterized in that**:
said treatment unit (7, 105, 145, 158, 162, 180, 202, 216, 223) has a treatment part main body (103) for supporting said treatment part (6, 102);
said locking member (108, 127, 211) is a restricting member that is further adapted to restrict advancing or retracting movement of said treatment unit (7, 105, 145, 158, 162, 180, 202, 216, 223) inside said channel (5, 57, 107); and
said treatment unit driving mechanism (110, 154, 158, 178, 196) is an advance/retract driving mechanism for advancing or retracting said treatment unit (7, 105, 145, 158, 162, 180, 202, 216, 223) between the base end side and the front end side of said channel (5, 57, 107), and for driving said treatment part (6, 102) by advancing or retracting said treatment part (6, 102) relative to said channel (5, 57, 107) and said treatment part main body (103) in a state where the advance or retraction of said treatment part main body (103) is restricted by said restricting member (108, 127, 211).

10. An endoscope treatment system according to claim 9, **characterized in that** said restricting member (108, 127, 211) is provided with a convex part (136) that can recess inward in the radial direction of said channel (5, 57, 107), and a recessing mechanism (137) for recessing said convex part (136).

11. An endoscope treatment system according to claim 9, **characterized in that** a blocking mechanism (131, 133) is provided for blocking the action of said treatment part (6, 102) during advancing and retracting movement of said treatment unit (7, 105, 145, 158, 162, 180, 202, 216, 223).

12. An endoscope treatment system according to claim 9, **characterized in that** said treatment unit (7, 105, 145, 158, 162, 180, 202, 216, 223) is formed so that it is detachable with said advance/retract driving mechanism (110, 154, 158, 178, 196).

13. An endoscope treatment system according to claim 12, **characterized in that**:
said advance/retract driving mechanism (110, 154, 158, 178, 196) is provided with an operation line(13, 117, 142, 155, 181, 197, 198) that can advance and retract within said channel (5, 57, 107) and is connected to said treatment unit (7, 105, 145, 158, 162, 180, 202, 216, 223), and an advance/retract mechanism (118) for advancing and retracting said operation line (13, 117, 142, 155, 181, 197, 198);
said treatment unit (7, 105, 145, 158, 162, 180, 202, 216, 223) is provided with a treatment part driving mechanism (8, 110, 126, 154, 158, 178, 196) that is supported by said treatment part main body (103) and connected to a procedure line part (120), and is for communicating the advancing/retracting action of said operation line (13, 117, 142, 155, 181, 197, 198) with respect to said treatment part main body (103), to said treatment part (7, 105, 145, 158, 162, 180, 202, 216, 223); and
there is provided a locking mechanism (127) that can rotate around its center axis under the condition where said treatment part driving mechanism (8, 110, 126, 154, 158, 178, 196) and said operation line (13, 117, 142, 155, 181, 197, 198) are mutually locked.

14. An endoscope treatment system according to claim 13, **characterized in that** the outer peripheral surface of said operation line (13, 117, 142, 155, 181, 197, 198) may be covered with a helical member (144, 185).

15. An endoscope treatment system according to claim 9, **characterized in that** the endoscope treatment system further comprises an extension part (163) that comes into contact with said restricting member (108, 127, 211), is connected to said treatment unit (7, 105, 145, 158, 162, 180, 202, 216, 223)and said advance/retract driving mechanism (110, 154, 158, 178, 196), and is formed to have a length capable of projecting said treatment unit (7, 105, 145, 158, 162, 180, 202, 216, 223) out from the front end of said channel (5, 57, 107).

16. An endoscope treatment system according to claim 10, **characterized in that** the endoscope treatment system further comprises a projecting mechanism (170) capable of projecting said convex part (136) along with a portion of said channel (5, 57, 107) outward from the front end of said channel (5, 57, 107).

## Patentansprüche

1. Endoskopbehandlungssystem (4, 41, 48, 55, 101, 140, 153, 161, 167, 177, 195, 210, 221), aufweisend:
ein Endoskop (3, 106, 141, 168, 187) mit einem Einführteil (2, 111, 176);
einen Kanal (5, 57, 107), der entlang des Einführteils (2, 111, 176) vorgesehen ist;
eine Behandlungseinheit (7, 105, 145, 158, 162, 180, 202, 216, 223), welche in den Kanal (5, 57, 107) einführbar ist und an ihrem vorderen Ende ein Behandlungsteil (6, 102) hat; und
einen Behandlungseinheitantriebsmechanismus (8, 110, 126, 154, 158, 178, 196) zum Bewegen der Behandlungseinheit (7, 105, 145, 158, 162, 180, 202, 216, 223) in dem Kanal (5, 57, 107),
**dadurch gekennzeichnet, dass** es weiterhin ein Verriegelungsbauteil (10, 56, 108, 127, 211) aufweist, das an einer Seite eines vorderen Endes des Kanals (5, 57, 107) in dem Einführteil (2, 11, 176) angeordnet ist und einen Betrag, um den die Behandlungseinheit (7, 105, 145, 158, 162, 180, 202, 216, 223) von dem vorderen Ende des Kanals (5, 57, 107) vorragt, zu begrenzen vermag, indem es die Behandlungseinheit (7, 105, 145, 158, 162, 180, 202, 216, 223) verriegelt, wenn die Behandlungseinheit (7, 105, 145, 158, 162, 180, 202, 216, 223) vorwärts bewegt wird.

2. Endoskopbehandlungssystem nach Anspruch 1,
**dadurch gekennzeichnet, dass**:
der Behandlungseinheitsantriebsmechanismus (8, 110, 126, 154, 158, 178, 196) ein Vorschub-Antriebsmechanismus ist, um die Behandlungseinheit (7, 105, 145, 158, 162, 180, 202, 216, 223) in dem Kanal (5, 57, 107) in Richtung des vorderen Endes des Kanals (5, 57, 107) vorwärts zu bewegen.

3. Endoskopbehandlungssystem nach Anspruch 2,
**dadurch gekennzeichnet, dass** die Behandlungseinheit (7, 105, 145, 158, 162, 180, 202, 216, 223) mit einem Behandlungsteilantriebsmechanismus (25, 126, 191, 206), um die Vorschub/Zurückzieh-Antriebskraft aufzunehmen und das Behandlungsteil (6, 102) anzutreiben, und mit einem Verbindungsmechanismus (26, 132, 147, 225) zur Verbindung des vorderen Endes des Vorschub-Antriebsmechanismus (8, 110, 126, 154, 158, 178, 196) und der Basisendseite des Behandlungsteilantriebsmechanismus (25, 126, 191, 206) versehen ist, indem der Vorschub-Antriebsmechanismus (8, 110, 126, 154, 158, 178, 196) in einem Zustand vorwärts geschoben wird, in welchem die Behandlungseinheit (7, 105, 145, 158, 162, 180, 202, 216, 223) von dem Verriegelungsbauteil (10, 56, 108, 127, 211) verriegelt ist.

4. Endoskopbehandlungssystem nach Anspruch 3,
**dadurch gekennzeichnet, dass**:
der Vorschub-Antriebsmechanismus (8, 110, 126, 154, 158, 178, 196) mit einer rohrförmigen Betätigungsröhre (12, 50) und einer Betätigungsleitung (13, 117, 142, 155, 181, 197, 198) versehen ist, der angeordnet ist, um einen Vorschub und eine Zurückziehung entlang der Axialrichtung der Betätigungsröhre (12, 50) zu ermöglichen und der zur Verbindung mit dem Behandlungsteil-antriebsmechanismus (25, 126, 191, 206) durch den Verbindungsmechanismus (26, 132, 147, 225) dient; und
dass die Behandlungseinheit (7, 105, 145, 158, 162, 180, 202, 216, 223) mit einem Betätigungsröhrenverriegelungsteil (30, 52) versehen ist, um die Betätigungsröhre (12, 50) zu verriegeln.

5. Endoskopbehandlungssystem nach Anspruch 4,
**dadurch gekennzeichnet, dass** der Verbindungsmechanismus (26, 132, 147, 225) versehen ist mit:
einem Kupplungshauptkörper (31) in Form eines Stabs, der von der Basisendseite der Behandlungseinheit (7, 105, 145, 158, 162, 180, 202, 216, 223) aus vorsteht;
einem runden Bauteil (32), das an der Seitenkante der Betätigungsröhre (12, 50) des Kupplungshauptkörpers (31) angeordnet ist und einen Durchmesser hat, der größer als der Außendurchmesser des Kupplungshauptkörpers (31) ist;
einem konkaven Teil (33), das an dem vorderen Ende der Betätigungsleitung (13, 117, 142, 155, 181, 197, 198) angeordnet und für einen Eingriff mit dem runden Bauteil (32) vorgesehen ist; und
einem Schlitz (35) zum Führen des runden Bauteils (32) in das konkave Teil (33), wobei die Breite seiner Öffnung kleiner als der Durchmesser des runden Bauteils (32) ist und der elastisch verformbar ist, um das Einführen des runden Bauteils (32) zu ermöglichen.

6. Endoskopbehandlungssystem nach Anspruch 2,
**dadurch gekennzeichnet, dass** das Verriegelungsbauteil (10, 56, 108, 127) bezüglich des vorderen Endes des Kanals (5, 57, 107) abnehmbar ist.

7. Ein Endoskopbehandlungssystem nach Anspruch 4,
**dadurch gekennzeichnet, dass** ein elastisches Bauteil (45), das sich in axialer Richtung frei ausdehnen und zusammenziehen kann, zwischen dem Betätigungsröhrenverriegelungsteil (30, 52) und dem Verriegelungsbauteil (10, 56, 108, 127, 211) angeordnet ist.

8. Endoskopbehandlungssystem nach Anspruch 4,
**dadurch gekennzeichnet, dass**:
wenigstens entweder ein röhrenseitiges konkaves Teil (51A) oder ein röhrenseitiges konvexes Teil (51B) an dem vorderen Ende der Betätigungsröhre (12, 50) angeordnet ist; und
ein verriegelungsseitiges konvexes Teil (52A) und/oder ein verriegelungsseitiges konkaves Teil (52B), das mit dem röhrenseitigen konkaven Teil (51A) und/oder dem röhrenseitigem konvexen Teil (51B) in Eingriff gelangen kann, an dem Betätigungsröhrenverriegelungsteil (30, 52) angeordnet ist.

9. Endoskopbehandlungssystem nach Anspruch 1,
**dadurch gekennzeichnet, dass**:
die Behandlungseinheit (7, 105, 145, 158, 162, 180, 202, 216, 223) einen Behandlungsteilhauptkörper (103) zum Lagern des Behandlungsteils (6, 102) hat;
das Verriegelungsteil (108, 127, 211) ein Begrenzungsteil ist, welches weiterhin eine Vorschub- oder Zurückziehbewegung der Behandlungseinheit (7, 105, 145, 158, 162, 180, 202, 216, 223) innerhalb des Kanals (5, 57, 107) zu begrenzen vermag; und
der Behandlungseinheitantriebsmechanismus (110, 154, 158, 178, 196) ein Vorschub/Zurückziehantriebsmechanismus ist, um die Behandlungseinheit (7, 105, 145, 158, 162, 180, 202, 216, 223) zwischen der Basisendseite und der Vorderendseite des Kanals (5, 57, 107) relativ zu dem Kanal (5, 57, 107) und dem Behandlungsteilhauptkörper (103) in einem Zustand vorzuschieben oder zurückzuziehen, in welchem das Vorschieben oder Zurückziehen des Behandlungsteilhauptkörpers (103) von dem Begrenzungsteil (108, 127, 211) begrenzt ist.

10. Endoskopbehandlungssystem nach Anspruch 9,
**dadurch gekennzeichnet, dass** das Begrenzungsteil (108, 127, 211) mit einem konvexen Teil (136) versehen ist, welches sich in Radialrichtung des Kanals (5, 57, 107) nach innen aufweiten kann, sowie einem Aufweitungsmechanismus (137) zum Aufweiten des konvexen Teils (136).

11. Endoskopbehandlungssystem nach Anspruch 9,
**dadurch gekennzeichnet, dass** ein Blockiermechanismus (131, 133) vorgesehen ist, um während der Vorschub- und Zurückziehbewegung der Behandlungseinheit (7, 105, 145, 158, 162, 180, 202, 216, 223) die Wirkweise des Behandlungsteils (6, 102) zu blockieren.

12. Endoskopbehandlungssystem nach Anspruch 9,
**dadurch gekennzeichnet, dass** die Behandlungseinheit (7, 105, 145, 158, 162, 180, 202, 216, 223) so ausgebildet ist, dass sie mit dem Vorschub/Zurückziehantriebsmechanismus (110, 154, 158, 178, 196) abnehmbar ist.

13. Ein Endoskopbehandlungssystem nach Anspruch 12,
**dadurch gekennzeichnet, dass**:
der Vorschub/Zurückziehantriebsmechanismus (110, 154, 158, 178, 196) mit einer Betätigungsleitung (13, 117, 142, 155, 181, 197, 198), die sich innerhalb des Kanals (5, 57, 107) vor und zurück bewegen kann und die mit der Behandlungseinheit (7, 105, 145, 158, 162, 180, 202, 216, 223) verbunden ist und mit einem Vorschub/Zurückziehmechanismus (118) versehen ist, um die Betätigungsleitung (13, 117, 142, 155, 181, 197, 198) vor und zurück zu bewegen;
wobei die Behandlungseinheit (7, 105, 145, 158, 162, 180, 202, 216, 223) mit einem Behandlungsteilantriebsmechanismus (8, 110, 126, 154, 158, 178, 196) versehen ist, der von dem Behandlungsteilhauptkörper (103) gelagert wird und mit einem Handlungsleitungsteil (120) verbunden ist und zur Übertragung der Vorschub/Zurückziehwirkung der Betätigungsleitung (13, 117, 142, 155, 181, 197, 198) bezüglich des Behandlungsteilhauptkörpers (103) an das Behandlungsteil (7, 105, 145, 158, 162, 180, 202, 216, 223) dient; und
dass ein Verriegelungsmechanismus (127) vorgesehen ist, der in einem Zustand um seine Mittelachse rotieren kann, in welchem der Behandlungsteilantriebsmechanismus (8, 110, 126, 154, 158, 178, 196) und die Betätigungsleitung (13, 117, 142, 155, 181, 197, 198) miteinander verriegelt sind.

14. Endoskopbehandlungssystem nach Anspruch 13,
**dadurch gekennzeichnet, dass** die äußere Umfangsoberfläche der Betätigungsleitung (13, 117, 142, 155, 181, 197, 198) von einem Schraubenwicklungsteil (144, 185) bedeckt sein kann.

15. Endoskopbehandlungssystem nach Anspruch 9,
**dadurch gekennzeichnet, dass** das Endoskopbehandlungssystem weiterhin ein Verlängerungsteil (163) aufweist, das in Kontakt mit dem Begrenzungsteil (108, 127, 211) gelangt, mit der Behandlungseinheit (7, 105, 145, 158, 162, 180, 202, 216, 223) und dem Vorschub/Zurückziehantriebsmechanismus (110, 154, 158, 178, 196) verbunden ist und mit einer derartigen Länge ausgebildet ist, dass die Behandlungseinheit (7, 105, 145, 158, 162, 180, 202, 216, 223) von dem vorderen Ende des Kanals (5, 57, 107) heraus gestreckt wird.

16. Endoskopbehandlungssystem nach Anspruch 10,
**dadurch gekennzeichnet, dass** das Endoskopbehandlungssystem weiterhin einen Vorstreckmechanismus (170) aufweist, der in der Lage ist, das konvexe Teil (136) zusammen mit einem Teil des Kanals (5, 57, 107) aus dem Vorderende des Kanals (5, 57, 107) nach außen vorstehen zu lassen.

## Revendications

1. Système de traitement endoscopique (4, 41, 48, 55, 101, 140, 153, 161, 167, 177, 195, 210, 221) comprenant :
un endoscope (3, 106, 141, 168, 187) comportant une partie insérée (2, 111, 176) ;
un canal (5, 57, 107) prévu le long de ladite partie insérée (2, 111, 176) ;
une unité de traitement (7, 105, 145, 158, 162, 180, 202, 216, 223) qui peut être insérée dans ledit canal (5, 57, 107) et comporte une partie de traitement (6, 102) au niveau de son extrémité avant ; et
un mécanisme d'entraînement d'unité de traitement (8, 110, 126, 154, 158, 170, 196) destiné à déplacer ladite unité de traitement (7, 105, 145, 158, 162, 180, 202, 216, 223) à l'intérieur dudit canal (5, 57, 107) ;
**caractérisé en ce qu'**il comprend de plus un élément de blocage (10, 56, 108, 127, 211) prévu sur un côté d'une extrémité avant du canal (5, 57, 107) dans ladite partie insérée (2, 111, 176), et adapté pour limiter une quantité de saillie de l'unité de traitement (7, 105, 145, 158, 162, 180, 202, 216, 223) de l'extrémité avant du canal (5, 57, 107) en bloquant ladite unité de traitement (7, 105, 145, 158, 162, 180, 202, 216, 223) lorsque ladite unité de traitement (7, 105, 145, 158, 162, 180, 202, 216, 223) est en cours de déplacement vers l'avant.

2. Système de traitement endoscopique selon la revendication 1, **caractérisé en ce que** :
ledit mécanisme d'entraînement d'unité de traitement (8, 110, 126, 154, 158, 170, 196) est un mécanisme d'entraînement d'avance destinée à faire avancer ladite unité de traitement (7, 105, 145, 158, 162, 180, 202, 216, 223) à l'intérieur dudit canal (5, 57, 107) vers l'extrémité avant dudit canal (5, 57, 107).

3. Système de traitement endoscopique selon la revendication 2, **caractérisé en ce que** ladite unité de traitement (7, 105, 145, 158, 162, 180, 202, 216, 223) est munie d'un mécanisme d'entraînement de partie de traitement (25, 126, 191, 206) destiné à recevoir une force d'entraînement d'avance/de recul et à entraîner ladite partie de traitement (6, 102), et d'un mécanisme de connexion (26, 132, 147, 225) destiné à connecter l'extrémité avant dudit mécanisme d'entraînement d'avance (8, 110, 126, 154, 158, 170, 196) et le côté d'extrémité de base dudit mécanisme d'entraînement de partie de traitement (25, 126, 191, 206), en faisant avancer ledit mécanisme d'entraînement d'avance (8, 110, 126, 154, 158, 170, 196) sous la condition dans laquelle ladite unité de traitement (7, 105, 145, 158, 162, 180, 202, 216, 223) est bloquée par ledit élément de blocage (10, 56, 108, 127).

4. Système de traitement endoscopique selon la revendication 3, **caractérisé en ce que** :
ledit mécanisme d'entraînement d'avance (8, 110, 126, 154, 158, 170, 196) est muni d'un tube d'actionnement tubulaire (12, 50) et d'un câble d'actionnement (13, 117, 142, 155, 181, 197, 198) qui est disposé pour permettre l'avance et le retrait le long de la direction axiale dudit tube d'actionnement (12, 50) et est destiné à se connecter audit mécanisme d'entraînement de partie de traitement (25, 126, 191, 206) par ledit mécanisme de connexion (26, 132, 147, 225) ; et
ladite unité de traitement (7, 105, 145, 158, 162, 180, 202, 216, 223) est munie d'une partie de blocage de tube d'actionnement (30, 52) destinée à bloquer ledit tube d'actionnement (12, 50).

5. Système de traitement endoscopique selon la revendication 4, **caractérisé en ce que** ledit mécanisme de connexion (26, 132, 147, 225) est muni :
d'un corps principal de couplage (31) formé selon la forme d'une tige faisant saillie hors du côté d'extrémité de base de ladite unité de traitement (7, 105, 145, 158, 162, 180, 202, 216, 223) ;
d'un élément rond (32) disposé sur le bord latéral du tube d'actionnement (12, 50) dudit corps principal de couplage (31) et présentant un diamètre qui est supérieur au diamètre externe dudit corps principal de couplage (31) ;
d'une partie concave (33) qui est disposée sur l'extrémité avant dudit câble d'actionnement (13, 117, 142, 155, 181, 197, 198) et est destinée à se mettre en prise avec ledit élément rond (32) ; et
d'une fente (35) destinée à guider ledit élément rond (32) dans ladite partie concave (33), dans laquelle la largeur de l'ouverture est inférieure au diamètre dudit élément rond (32) et qui peut être élastiquement déformée pour permettre l'insertion dudit élément rond (32).

6. Système de traitement endoscopique selon la revendication 2, **caractérisé en ce que** ledit élément de blocage (10, 56, 108, 127) est détachable de l'extrémité avant dudit canal (5, 57, 107).

7. Système de traitement endoscopique selon la revendication 4, **caractérisé en ce qu'**un élément élastique (45) qui peut être librement dilaté et contracté dans la direction axiale est disposé entre ladite partie de blocage de tube d'actionnement (30, 52) et ledit élément de blocage (10, 56, 108, 127, 211).

8. Système de traitement endoscopique selon la revendication 4, **caractérisé en ce que** :
au moins l'une ou l'autre parmi une partie concave du côté du tube (51A) et une partie convexe du côté du tube (51B) est disposée sur l'extrémité avant dudit tube d'actionnement (12, 50) ; et
au moins l'une ou l'autre parmi une partie convexe côté blocage (52A) et une partie concave côté blocage (52B) qui peut se mettre en prise avec au moins l'une ou l'autre parmi la partie concave du côté du tube (51A) et la partie convexe du côté du tube (51B) est disposée sur ladite partie de blocage de tube d'actionnement (30, 52).

9. Système de traitement endoscopique selon la revendication 1, **caractérisé en ce que** :
ladite unité de traitement (7, 105, 145, 158, 162, 180, 202, 216, 223) comporte un corps principal de partie de traitement (103) destiné à supporter ladite partie de traitement (6, 102) ;
ledit élément de blocage (108, 127, 211) est un élément de limitation qui est de plus adapté pour limiter le mouvement d'avance ou de recul de ladite unité de traitement (7, 105, 145, 158, 162, 180, 202, 216, 223) à l'intérieur dudit canal (5, 57, 107) ; et
ledit mécanisme d'entraînement d'unité de traitement (110, 154, 158, 178, 196) est un mécanisme d'entraînement d'avance/de recul destiné à avancer ou à reculer ladite unité de traitement (7, 105, 145, 158, 162, 180, 202, 216, 223) entre le côté d'extrémité de base et le côté d'extrémité avant dudit canal (5, 57, 107), et destiné à entraîner ladite partie de traitement (6, 102) en avançant ou en reculant ladite partie de traitement (6, 102) par rapport audit canal (5, 57, 107) et audit corps principal de partie de traitement (103) dans un état dans lequel l'avance ou le recul dudit corps principal de partie de traitement (103) est limité par ledit élément de limitation (108, 127, 211).

10. Système de traitement endoscopique selon la revendication 9, **caractérisé en ce que** ledit élément de limitation (108, 127, 211) est muni d'une partie convexe (136) qui peut s'encastrer vers l'avant dans le sens radial dudit canal (5, 57, 107), et d'un mécanisme d'encastrement (137) pour l'encastrement de ladite partie convexe (136).

11. Système de traitement endoscopique selon la revendication 9, **caractérisé en ce qu'**un mécanisme de blocage (131, 133) est prévu pour bloquer l'action de ladite partie de traitement (6, 102) pendant le mouvement d'avance et de recul de ladite unité de traitement (7, 105, 145, 158, 162, 180, 202, 216, 223).

12. Système de traitement endoscopique selon la revendication 9, **caractérisé en ce que** ladite unité de traitement (7, 105, 145, 158, 162, 180, 202, 216, 223) est formée de sorte qu'elle est détachable avec ledit mécanisme d'entraînement d'avance/de recul (110, 154, 158, 178, 196).

13. Système de traitement endoscopique selon la revendication 12, **caractérisé en ce que** :
ledit mécanisme d'entraînement d'avance/de recul (110, 154, 158, 178, 196) est muni d'un câble d'actionnement (13, 117, 142, 155, 181, 197, 198) qui peut avancer et reculer à l'intérieur dudit canal (5, 57, 107) et est connecté à ladite unité de traitement (7, 105, 145, 158, 162, 180, 202, 216, 223), et d'un mécanisme d'avance/de recul (118) destiné à avancer et à reculer ledit câble d'actionnement (13, 117, 142, 155, 181, 197, 198) ;
ladite unité de traitement (7, 105, 145, 158, 162, 180, 202, 216, 223) est munie d'un mécanisme d'entraînement de partie de traitement (8, 110, 126, 154, 158, 170, 196) qui est supporté par ledit corps principal de partie de traitement (103) et connecté à une partie de câble de procédure (120), et est destinée à communiquer l'action d'avance/de recul dudit câble d'actionnement (13, 117, 142, 155, 181, 197, 198) par rapport audit corps principal de partie de traitement (103), à ladite partie de traitement (7, 105, 145, 158, 162, 180, 202, 216, 223) ; et
un mécanisme de blocage (127) est prévu, lequel peut tourner autour de son axe central dans la condition dans laquelle ledit mécanisme d'entraînement de partie de traitement (8, 110, 126, 154, 158, 170, 196) et ledit câble d'actionnement (13, 117, 142, 155, 181, 197, 198) sont mutuellement bloqués.

14. Système de traitement endoscopique selon la revendication 13, **caractérisé en ce que** la surface périphérique externe dudit câble d'actionnement (13, 117, 142, 155, 181, 197, 198) peut être couverte par un élément hélicoïdal (144, 185).

15. Système de traitement endoscopique selon la revendication 9, **caractérisé en ce que** le système de traitement endoscopique comprend en outre une partie d'extension (163) qui vient en contact avec ledit élément de limitation (108, 127, 211), est connecté à ladite unité de traitement (7, 105, 145, 158, 162, 180, 202, 216, 223) et audit mécanisme d'entraînement d'avance/de recul (110, 154, 158, 178, 196) et est formé pour présenter une longueur capable de faire saillir ladite unité de traitement (7, 105, 145, 158, 162, 180, 202, 216, 223) hors de l'extrémité avant dudit canal (5, 57, 107).

16. Système de traitement endoscopique selon la revendication 10, **caractérisé en ce que** le système de traitement endoscopique comprend en outre un mécanisme de saillie (170) capable de faire saillir ladite partie convexe (136) avec une partie dudit canal (5, 57, 107) vers l'extérieur de l'extrémité avant dudit canal (5, 57, 107).
